# EUROPEAN PATENT SPECIFICATION

(11) **EP 4 531 820 B1**
(45) Date of publication and mention of the grant of the patent: **10.09.2025**
(21) Application number: 22732463.9
(22) Date of filing: 31.05.2022
(51) Int. Cl.: A61K 9/70, A61L 29/08, A61M 25/00

(54) **SUBSTRATE FOR IMPLANTABLE MICROELECTRODES, ELECTRODE ELEMENT AND ELECTRODE SYSTEM, AND METHOD OF MANUFACTURING THEREOF**
SUBSTRAT FÜR IMPLANTIERBARE MIKROELEKTRODEN, ELEKTRODENELEMENT UND ELEKTRODENSYSTEM SOWIE VERFAHREN ZUR HERSTELLUNG DERSELBEN
SUBSTRAT POUR MICROELECTRODES IMPLANTABLES, ELEMENT D'ELECTRODE ET SYSTEME D'ELECTRODES, ET LEUR PROCEDE DE FABRICATION

(43) Date of publication of application: 09.04.2025
(73) Proprietor: NTT Research, Inc., Sunnyvale, CA 94085 (US)
(72) Inventor: TESHIMA, Tetsuhiko, 80802 München (DE); WOLFRUM, Bernhard, 85716 München (DE); ZURITA, Francisco, 81541 München (DE); DEL DUCA, Fulvia, 81547 Munich (DE); HIENDLMEIER, Lukas, 94348 Rinkam (DE)
(74) Representative: Appelt, Christian W.
(86) International application number: PCT/EP2022/064753
(87) International publication number: WO 2023/232229

(56) References cited:
- WO-A1-2011/058171
- WO-A2-02/087642
- US-B2- 8 809 411

## Description

### TECHNICAL FIELD

The present disclosure relates to a substrate for implantable microelectrodes and an electrode element comprising the substrate and an electrode system comprising the electrode element.

### BACKGROUND

In current practice, implantable microelectrodes are used for neuroscience, for alleviating symptoms of neurological diseases, such as Parkinson's disease or epilepsy, or for restoring body functions after injury. Implantable microelectrodes are brought into the body of the patient via surgery and are used for recording from or stimulating the neural target tissue and nerves. Examples of implantable microelectrodes are stimulation electrodes that are used for deep brain stimulation. Other examples are cuff electrodes used for interfacing nerves and electrodes used for electrocorticography.

US2021/0270764 A1 discloses a microelectrode having a layered structure, including a layer containing a polymer compound having an aromatic ring (polymer compound layer) and a layer containing a conductive material (conductive layer), and the microelectrode has a three-dimensional curved shape. Such a microelectrode having a small dimension (nano- and micro-meter range) would be generally difficult to handle during an operation, for example, when implanting the microelectrode into a tissue. A further challenge may be accurate fabrications of such three-dimensional structures of the microelectrode.

WO 02/087642 A2 discloses an adhesive composition containing both a hydrophobic phase and a hydrophilic phase, wherein the hydrophobic phase is composed of a crosslinked hydrophobic polymer composition and the hydrophilic phase is a water-absorbent blend of a hydrophilic polymer and a complementary oligomer capable of crosslinking the hydrophilic polymer through hydrogen bonding, ionic bonding, and/or covalent bonding.

US 8,809,411 B2 discloses a coating formulation for preparing a hydrophilic coating, wherein the hydrophilic coating formulation comprises a hydrophilic polymer, a supporting polymer comprising a backbone and at least 2 reactive moieties capable of undergoing polymerization reactions, a Norrish Type I photoinitiator and a Norrish Type II photoinitiator.

WO 20 11/058171 A1 discloses a coating composition comprising a hydrophilic polymer and a copolymer comprising a hydrophilic monomer and a monomer according to the structure X, wherein R = End group R1= Connecting group Z = Polymerizable group and to medical devices, preferably a catheter, comprising the coating.

### SUMMARY OF INVENTION

The technical problem to be solved may be formulated to provide a substrate for an implantable microelectrode and an electrode element/system including the substrate, which provides a stable, reliable, and controlled structure and may realize more accurate handling by a user.

A substrate for an implantable microelectrode according to the present disclosure comprises a substrate base layer (1) being a polymer compound layer. The substrate is preferably a film or a flexible polymer compound(s) for forming and/or mounting an implantable microelectrode thereon and the substrate is preferably electrically insulating material. The implantable microelectrode may be for example a cuff electrode that is able to interface nerves with a diameter of about 50 to 500 µm. During implantation, the mechanical actuation of the implantable microelectrode according to the present disclosure can be initiated by an external stimulus, i.e. contacting liquid comprising water, which may allow the implantable microelectrode to deform and curve, bend, or fold itself around e.g. a nerve for forming a close and stable interface with the biological tissue. The implantable microelectrode may have a small area being electrically conductive for contacting a biological sample, and the area may be typically in a range of micrometers and/or millimeters. The implantable microelectrode may be used, for example, for recording signals from the nerve tissue in the mV range or V range, or for stimulation of the nerve tissue with current pulses in the µA to mA range or with voltage pulses in the mV or V range. The substrate base layer (1) has a thickness (d₁) in the z-direction and comprises a first surface (1-1) and a second surface (1-2), each of the first surface (1-1) and the second surface (1-2) extending in the x-direction and the y-direction (and/or in the xy-plane), the second surface (1-2) being opposite to the first surface (1-1) in the z-direction. The x-, y-, z- axes and/or directions are of the three-dimensional rectangular coordinate system, which are orthogonal to each other.

The substrate base layer (1) consists of and/or is made of a hybrid material of (or comprising) a hydrophobic part (or element or portion or component or compound) and a hydrophilic part (or element or portion or component or compound). The hybrid material (i.e. the substrate base layer) is based on (and/or is cured from and/or is obtained and/or is obtainable by curing/polymerizing/photo-polymerizing) a mixture (10) (and/or a resin mixture and/or a pre-polymer solution) being photo-curable. The mixture comprises (a) a photoinitiator being able to be activated by exposure to light, (b) a first component (or a first molecule or a first compound) and (c) a second component (or a second molecule or a second compound). The molecular weight (M_{wa}) and/or the chain length of the first component is larger than the molecular weight (M_{wb}) and/or the chain length of the second component, preferably such that a diffusion coefficient and/or a mobility of the first component is smaller than a diffusion coefficient and/or a mobility of the second component in solution. The hydrophobic part may comprise the first component being polymerized and the hydrophilic part may comprise the second component being polymerized. The first component comprises a first hydrophobic monomer or a first hydrophobic oligomer or a first hydrophobic polymer or a first hydrophobic copolymer, wherein the first hydrophobic monomer or oligomer or polymer or copolymer of the first component comprises a first functional group, wherein the first functional group is able to be activated by the activated photoinitiator, so that the first component (and/or a plurality of the first components) is able to be polymerized through the first functional groups (more specifically by cross-linking of the first functional groups). The second component comprises a hydrophilic monomer or a hydrophilic oligomer or a hydrophilic polymer or a hydrophilic copolymer, wherein the hydrophilic monomer or oligomer or polymer or copolymer may be able to form a hydrogel. The hydrophilic monomer or oligomer or polymer or copolymer comprises a second functional group, wherein the second functional group is able to be activated by the activated photoinitiator, so that the second component (and/or a plurality of the second components) is able to be polymerized through the second functional groups (more specifically by cross-linking of the second functional groups), and so that the first component(s) and the second component(s) are able to be co-polymerized through the first functional groups and the second functional groups (more specifically by cross-linking of and/or via the first and second functional groups). The mixture may comprise a plurality of the first components and a plurality of the second components.

The substrate base layer may be obtained or obtainable by photopolymerization (and/or curing) of the mixture, more specifically, by exposing the mixture to light such as UV light and/or visible and/or infrared light, and more preferably, light with a wavelength in the range of 200 - 400 nm. The photopolymerization is a light-induced reaction, which converts a liquid mixture of one or more monomers and/or oligomers and/or polymers into a solid and/or hardened polymer. The reaction may require the use of an appropriate photoinitiator, which is a light sensitive molecule that produces active species or radicals upon irradiation with UV, visible, or infrared light, i.e. a photoinitiator is able to be activated by exposure to light to form a radical. The activated photoinitiator (or radicals or active species produced by the activated photoinitiator) may active the first/second functional group(s) of the (plurality of) first/second component(s). The (plurality of) first/second component(s) is/are polymerized through the activated first/second functional group(s) to form a polymer network. A monomer in general comprises a single unit of a certain chemical compound to be polymerized and is a chemical unit before polymerization. An oligomer comprises more than one unit of the same or structurally similar chemical compounds (and/or monomers), i.e. a number of units of the same or structurally similar chemical compound of more than one and less than 30. A polymer comprises a higher number of units of the same or structurally similar chemical compounds (and/or monomers). The oligomer and polymer are therefore a chemical compound after polymerization and/or obtainable by polymerization of a plurality of chemical units (monomers). A hydrogel(s) represent(s) a cross-linked hydrophilic polymer network(s) that absorb water molecules from liquid including water molecules but do not dissolve in the liquid when brought into contact with the liquid including water molecules. They are a class of polymeric materials with the ability to take up and hold a certain amount of water, which is commonly associated with a volumetric swelling or expansion, and softening of the material producing a rubbery-like consistency and low interfacial tension parameters, e.g. low contact angle for water. Hydrogel (mechanical) characteristics mainly depend on the cross-linking density, the chemical composition of the polymeric chains, and the interaction between the network and surrounding liquids.

The polymer network of the polymer compound layer may be inhomogeneous especially in or along the z-direction. The first surface (1-1) is more hydrophilic than the second surface (1-2). More particularly, the first surface (1-1) is more hydrophilic than the second surface (1-2) and the second surface (1-2) is more hydrophobic than the first surface (1-1), so that the layer folds itself such that the second surface is concave (inner surface) and the first surface is convex (outer surface) of the folded layer, or the layer folds itself such that a curvature radius is defined in the z-direction and/or within the xz-plane. The folding behavior of the layer, e.g. a radius of curvature and direction of curvature, can be controlled by adjusting the thickness and the inhomogeneity characteristics of the substrate base layer. Since the folding is induced by exposure of the layer to a solvent such as water, the layer is self-folding or self-assembling. The substrate according to the present disclosure may have one or more of the following advantages: In case the layer is used for a microelectrode for bioelectronics, the microelectrode can wrap around the nerve tissue via self-folding during implantation without any further measures that otherwise would have to be carried out manually by the operating personnel. Hence, implantation of microelectrodes with the substrate (i.e. with the polymer compound layer) according to the present invention is performed more easily and less error-prone compared to conventional microelectrodes. Further, due to the self-assembly or self-folding capability of the layer according to the present invention, the microelectrode is brought into close contact with the nerve tissue, which improves the efficiency of signal transduction and stimulation pulses.

Preferably, the substrate base layer (1) comprises a first sub-region (R₁) including the first surface (1-1) and a second sub-region (R₂) including the second surface (1-2), the first sub-region (R₁) being different from the second sub-region (R₂), wherein the first sub-region (R₁) is separated from the second sub-region (R₂) in the z-direction, wherein the first sub-region (R₁) has a higher swelling capacity than the second sub-region (R₂). A swelling capacity of the first (second) sub-region may be obtained by an equation (m₁-m₀)/m₀, whereas m₀ is an initial mass of the first (second) sub-region (i.e. a mass of the first (second) sub-region before contacting the liquid), and m₁ is a mass of the first (second) sub-region after the first (second) sub-region has been contacted liquid including water molecules and/or swelled and/or saturated by components of the liquid. Since the mixture is cured such as to form an inhomogeneous polymer network, the cross-linked polymer network of the second sub-region may be richer on the first component in comparison to the cross-linked polymer network of the first sub-region. The cross-linked polymer network of the first sub-region may be richer on the second component or hydrophilic part in comparison to the cross-linked polymer network of the second sub-region. The first sub-region has a higher swelling capacity than the second sub-region, i.e. the cross-linked polymer network of the first sub-region is more hydrophilic and is able to take up a larger amount of hydrophilic solvent molecules, e.g. water or dimethyl sulfoxide molecules, than the cross-linked polymer network of the second sub-region. This results in a higher degree of volumetric swelling of the first sub-region than the second sub-region when the layer is exposed to a solvent such as water. Hence, controlling the composition and structure of the inhomogeneous polymer network of the layer and consequently, the structure, composition, and dimensions of the first and second sub-region of the layer allows controlling the folding behavior of the layer, e.g. radius of curvature.

Preferably, the substrate base layer (1) further comprises a band portion (2) and/or a stripe portion, the band portion (2) having a higher cross-linking density and/or a degree of cross-linking and/or a degree of conversion and/or than the second surface (1-2) and/or the second sub-region (R₂). The substrate base layer (1) has a length (l₁) in the x-direction and a width (w₁) in the y-direction. The band portion (2) extends for at least 50 % of the width (w₁) of the substrate base layer (1) in the y-direction, more preferably at least 80 % of the of width (w₁) of the substrate base layer (1) in the y-direction, more preferably at least 90 % of the width (w₁) of the layer (1) in the y-direction. The band portion (2) extends for at least 50 % of the entire extent of the substrate base layer (1) in the z-direction, more preferably at least 80 % of the entire extent of the layer (1) in the z-direction, more preferably at least 90 % of the entire extent of the layer (1) in the z-direction. The band portion (2) has a thickness (l₂) in the x-direction, the thickness (l₂) being smaller than the length (l₁) of the layer (1) in the x-direction. Preferably, the thickness (l₂) of the band portion (2) is less than 10 %, more preferably less than 5 % of the length (l₁) of the substrate base layer. The band portion accordingly has a longer side in the y-direction and a shorter side in the x-direction. A cross-linking density and/or a degree of cross-linking and/or a degree of conversion is the extent to which the components (including the first and/or second components) of the mixture react to form a polymer network, and/or is a number of cross-linked junctions (of the first and/or second functional groups) per unit volume. For example, the first functional group may be an acrylate group including a C=C double bond, and the cross-linking density is proportional to the ratio of C=C double bonds of the first functional groups that are converted into C-C single bonds by being exposed with light. The cross-linking density represents and/or is proportional to the density of the polymer network, which represents and/or is proportional to a mesh size of the polymer network. When the density of the polymer network is high, the mesh size of the polymer network is small, and when the density of the polymer network is low, the mesh size of the polymer network is large, wherein the mesh size may be a correlation or average length between crosslinks (i.e. cross-linked functional groups). The cross-linking density influences material (mechanical) characteristics of the polymer, such as stiffness or tensile strength, surface hardness, or swelling capacity. The band portion having a higher cross-linking density may have a higher stiffness than the other part of the substrate base layer (1), in particular, than the second sub-region (R₂). The band portion (2) and/or a stripe portion may be produced by prolonged exposure of the mixture (or the substrate base layer (1)) to light or by exposing the mixture to light having a higher irradiance at the band portion (2) and/or a stripe portion in comparison the other part of mixture.

The band portion (2) may have one of more of the following advantages: The band portion (2) may be especially advantageous for controlling the folding direction of the substrate (A) more accurately. Due to the higher cross-linking density, the band portion has a mechanical property being different from the other part of the substrate. The density of the polymer network inside of the band portion is higher than the other part of the substrate, especially than the second surface and/or the second sub-region (R2) other than the band portion. The band portion has accordingly a lower elasticity (i.e. is more rigid) than the other part of the substrate. When the substrate is submerged into liquid including water molecules, at least the first surface and/or the first sub-region (other than the band portion) is swelled by the components of the liquid and the substrate folds by itself, such that the second surface is the inner surface of the folded substrate. In this folding process, the band portion may serve as a frame restricting the deformation of the substrate in the y-direction, which is the main longitudinal direction (i.e. longer side) of the band portion, and the substrate accordingly folds up in the x-direction, i.e. in the direction (substantially) orthogonal to the main longitudinal direction of the band portion. More specifically, the folded substrate would have a folding-axis along the y-direction and the curvature radius of the folded substrate would be defined in the xz-plane.

Preferably, the length of the substrate base layer is longer than the width of the substrate base layer (1), and wherein the band portion (2) and/or the longer side of the band portion (2) extends parallel to the y-direction, or wherein the band portion (2) forms an angle with the y-direction, wherein the angle is more than 0° and less than or equal to 90°. Preferably, the substrate base layer (1) comprises a plurality of the band portions (2), and the plurality of the band portions (2) is spaced apart from each other in the x-direction.

Preferably, one or more active agents, selected from a group including drugs or labels, are comprised in or admixed to the mixture. Additionally or preferably, one or more active agents, selected from a group including drugs or labels, are comprised in or embedded in the first and/or the second component. The active agents are preferably able to diffuse out of the layer over time and are able to interact with biological tissue, for example, in case the layer is used for an implantable microelectrode for bioelectronics. The active agents can be used to fluorescently label cells in the biological tissue in the vicinity of the microelectrode or provide an anti-inflammatory effect.

Preferably, the substrate base layer (1) (and/or the hybrid material) is obtained and/or obtainable by frontal photopolymerization of the mixture, wherein the first surface (1-1) is produced and/or obtained and/or obtainable on the side from which the light is irradiated and/or applied. Frontal photopolymerization is a curing technique in which the mixture is exposed with light from/in only a single direction and/or from/in only a single side, more specifically only in the z-direction. Upon irradiating light, a polymerized front surface moves inside the uncured mixture in the z-direction, exclusively or mostly as long as the light is irradiated. The process relies on the penetration depth of light into a mixture or pre-polymer solution, wherein the penetration depth is defined by Lambert Beer's law and substantially exponentially decreases inside the curing mixture in a direction into which the light is irradiated, i.e. the z-direction. The deposited dose of light in the mixture depends on said penetration depth, which correlates with the cross-linking density at a certain depth inside the cured layer in the z-direction. The frontal photopolymerization process may be further advantageous for forming an inhomogeneous polymer network of the substrate base layer, which is reflected in an increased hydrophilicity of the first surface of the layer, from which the light is applied. The first sub-region of the layer comprising the first surface may have an increased swelling capacity compared to the second sub-region of the layer comprising the second surface.

Preferably, the substrate base layer (1) comprises a dent portion (7) at the second surface (1-2) or at the first surface (1-1) of the substrate base layer (1). Preferably, the dent portion (7) is a through-hole extending in the z-direction through the substrate base layer (1). The dent portion can be formed by irradiating a focused ion beam or a laser beam onto the second surface (1-2) and/or the first surface (1-1) of the substrate base layer (1). Alternatively, the dent portion (7) may be formed by applying a patterning including an uncured portion when curing the mixture. The formed dent portion may be further advantageous in that the dent portion can used as a microelectrode opening and/or an access passage for ions.

Preferably, the mixture further comprises (d) a third component and/or (e) one or more further components selected from a group including diluent or buffer components/free radical quenchers/ photosensitizers or UV absorbers/cross-linkers. The third component comprises a second hydrophobic monomer or a second hydrophobic oligomer or a second hydrophobic polymer or a second hydrophobic copolymer, wherein the second hydrophobic monomer or oligomer or polymer or copolymer is different from the first hydrophobic monomer or oligomer or polymer or copolymer. The second hydrophobic monomer or oligomer or polymer or copolymer of the third component comprises a third functional group, the third functional group being a different end-group than the first functional group and/or the second functional group. More specifically, the third functional group is attached to a position being different from the first functional group and/or the second functional group with respect to the monomer or oligomer or polymer or copolymer. The end-group is an extremity of a monomer or oligomer or polymer or copolymer, and may be a side chain (or group) and/or terminate (or terminal) group. The third functional group is able to be activated by the activated photoinitiator, so that the third component is able to be polymerized through the third functional groups and/or so that the first component and the second component and the third component are able to be co-polymerized through the first functional groups and the second functional groups and the third functional groups. For example, the third functional group being a different end-group than the first functional group means that the third component comprises one or more (meth)acrylated silicone polymers or (meth)acrylated silicone copolymers that contain one or two terminal (meth)acrylate groups on a linear polymeric chain while the first component has (meth)acrylate groups as side chains, or vice versa. This may result in a more heterogeneous mesh of the polymer network of the layer, i.e. a larger variation in mesh size or length in between the cross-linking junctions, which can affect the material characteristics of the layer such the tensile strength. Suitable examples for diluent or buffer components are one or more of amphiphilic acrylic acid isobornyl ester and/or isobornyl acrylate (IBA) and/or acrylic acid. The concentration of diluent or buffer components comprised in the mixture is preferably in a range of 0% to 89.9% w/w. A suitable example for a free radical quencher is 2,2,6,6-tetramethylpiperidine-1-oxyl (TEMPO), hydroquinone, 4-tert-Butylcatechol. The concentration of the free radical quencher comprised in the mixture is preferably in a range of 0% to 1% w/w. Suitable examples for photosensitizers or UV absorbers are 5-(benzoyl)-4-hydroxy-2-methoxybenzenesulfonic acid (HMBS) and/or 2-Isopropylthioxanthone (ITX). The concentration of the photosensitizers or UV absorbers comprised in the mixture is preferably in a range of 0% to 10% w/w. A suitable example for a cross-linker is tetraethylene glycol diacrylate (TEGDA)-

Preferably, the first functional group is an acrylate or methacrylate group. Preferably, the first component and/or the third component comprises an (meth)acrylated silicone polymer and/or an (meth)acrylated silicone copolymer, wherein the (meth)acrylated silicone polymer or the (meth)acrylated silicone copolymer contains one or two terminal acrylate groups and/or one or more acrylate groups at side chains, such as acryloxy-terminated ethyleneoxide dimethylsiloxane-ethyleneoxide ABA block copolymer (DBE-U22) and/or (acryloxypropyl)methylsiloxane dimethylsiloxane copolymer (UMS-182) and/or 3-acryloxy-2-hydroxypropoxypropyl terminated polydimethylsiloxane (DMS-U21) and/or acryloxypropyl T-structure siloxane (UTT-1012), (3-acryloxypropyl) trimethoxysilane (SIA0200) and/or vinyl-terminated polydimethylsiloxane (DMS-V05 and/or DMS-V21 and/or DMS V-22 and/or DMS V-25) and/or acryloxypropyl methysiloxane and/or dimethylsiloxane (UMS-182) and/or [(3-acryloxy-2-hydroxypropoxypropyl)methylsiloxane]/dimethylsiloxane (UCS-052). Each of DMS-V05, DMS-V21, DMS V-22, DMS V-25 is vinyl-terminated at both two ends, and its number (05, 21, 22 or 25) refers to the number of dimethylsiloxane. Preferably, the first component and/or the third component comprises polystyrene and/or a polyester and/or a polyurethane. Preferably, the second component comprises a hydrophilic acryl monomer such as 2-hydroxyethyl methacrylate (HEMA) and/or N,N-dimethylacrylamide (DMA). Preferably, the second component comprises Tetra(ethylene glycol) diacrylate (TEGDA) and/or poly(ethylene glycol) diacrylate (PEGDA), wherein a molecular weight per unit Mₙ of PEGDA may be Mₙ = 250 and/or 575 and/or 700. Alternatively, the first component and/or the second component and/or the photoinitiator or catalyst may be selected for polymerization via thiol-ene click chemistry.

Preferably, the concentration or content of the first component comprised in the mixture is preferably in a range of 5 to 95 % w/w, more preferably 20 % to 79.9 % w/w. The concentration of the second component comprised in the mixture is preferably in a range of 5 to 95 % w/w, more preferably 20 % to 79.9 % w/w. More preferably, the volumetric ratio of the first component to the second component in the mixture is 1:3 or 2:2 (1:1) or 3:1. The concentration of the photoinitiator added to the mixture is preferably in the range of 0.1-2 % w/w. A suitable example for the photonitiator is phenyl bis(2,4,6-trimethylbenzoyl)-phosphinoxide. The C-P bond in this molecule is cleaved via illumination with light of a wavelength below approximately 450 nm and the radicals 2,4,6-trimethylbenzoyl and phenylphosphonyl are generated. These radicals then activate the cross-linking of the first and second component at the (meth)acrylate functional groups. Further suitable examples for a photoinitiator are Bis(2,4,6-trimethylbenzoyl)phenylphosphine oxide, Blend of ethyl phenyl(2,4,6- trimethylbenzoyl)phosphinate and phenyl bis(2,4,6-trimethylbenzoyl)-phosphine oxide, 2,4,6-trimethylbenzoyl-diphenyl phosphine oxide, 2-Hydroxy-4-(2-hydroxyethoxy)-2-methylpropiophenone, Bis(2,4,6-Trimethylbenzoyl)phenylphosphine oxide, 2-Isopropylthioxanthone, thioxanthone derivative, Lithium phenyl-2,4,6-trimethylbenzoylphosphinate.

Preferably, the substrate (A) further comprises a protruding part (3) protruding from the second surface (1-2) and/or the first surface (1-1) of the substrate base layer (1) in the z-direction. The protruding part may have one or more of the following advantages: When the substrate (A) is in the folded-state, the protruding part can then be point out of the outer surface away from the folded or curved substrate or out of the inner surface towards inside of the folded or curved substrate. Since the protruding part is on the first or second surface of the self-folding substrate, the substrate for microelectrodes for bioelectronics can more closely and stably interface biological tissue such as nerves when the substrate is implanted.

Preferably, the protruding part (3) is rod-shaped and extends through the layer (1) in the z-direction, or the protruding part (3) is based on the mixture and is integrally formed with the substrate base layer (1). The protruding part, e.g. pillar or needle, may be fabricated from metal-nanoparticle ink or conductive-polymer ink by three-dimensional deposition techniques such as inkjet, aerosol-jet, or extrusion-based printing, or other deposition techniques suitable for producing micrometer-sized structures in large aspect-ratios. The substrate base layer may be then formed, so that the protruding part is disposed in or on the substrate base layer. Alternatively, the protruding part may be integrally formed with the substrate base layer (1). More specifically, the protruding part may be obtained by photopolymerization of the same mixture for the substrate base layer. The protruding part may be further advantageous in that the protruding part would be tightly incorporated into the layer, which may improve its mechanical stability. Preferably, the protruding part is at least partially electrically conductive and/or is an electrically conductive material, such as a metal or conductive polymer. The protruding part being electrically conductive may be serve as a part of an electrode for applying current/voltage to a biological sample and is further advantageous in that the protruding part increases a surface area of the electrode for contacting the biological sample. The protruding part may more efficiently access and/or penetrate into the biological sample such as a nerve fibre. Alternatively, the protruding part can be formed from a dielectric material that is partly covered with a conductive layer. The dielectric material can, for example, be likewise deposited by three-dimensional printing techniques. The protruding part may be electrically connectable from both surface sides of the substrate base layer.

Preferably, the substrate (A) further comprises an embedded part being embedded in the layer (1), wherein the embedded part is partially exposed to the outside of the substrate base layer (1). Preferably, the embedded part is rod-shaped and extends in the z-direction of the substrate base layer (1), and the embedded part has a first end and a second end, the second end being different from the first end, wherein the first end is embedded inside of the substrate base layer (1) and the second end is exposed to the outside of the substrate base layer (1), wherein the second end is disposed within the plane of the second surface (1-2) or between the second surface (1-2) and the first surface (1-1). Preferably, the embedded part is rod-shaped and extends in the z-direction of the substrate base layer (1), and the embedded part has a first end and a second end, the second end being different from the first end, wherein the embedded part further extends through the substrate base layer (1) and the first end is exposed to the outside of the layer, wherein the first end is disposed within the plane of the first surface (1-1) or between the second surface (1-2) and the first surface (1-1). Preferably, the embedded part is a through-via forming an electrically conductive connection between the first surface and the second surface. Preferably, the protruding part and/or the embedded part comprise(s) one or more particle(s). The particles may be Ni, Co, Pt, Ag and Fe nanoparticles, or Ag, Au, and Cu nanowires. Preferably, the embedded part is electrically conductive.

An electrode element according to the present disclosure comprises the above-described substrate and further comprises an electrically conductive layer deposited on at least a part of the first surface and/or a part of the second surface of the layer. Preferably, the electrically conductive layer is further deposited on the protruding part and/or on the embedded part. Preferably, the electrode element further comprises a further protruding part protruding from the electrically conductive layer in the z-direction, wherein the protruding part is preferably electrically conductive. Preferably, the electrically conductive layer is transparent with respect to at least a range of light wavelengths, preferably a range of light wavelengths for curing the mixture. Such light can pass through the transparent electrically conductive layer preferably without being absorbed. The electrically conductive layer may be formed from a transparent electrically conductive material such as a carbon sheet or conductive polymer or indium tin oxide (ITO). Preferably, the thickness of the electrically conductive layer may be adapted for appropriate transparency properties. The electrically conductive layer may either covers a portion of the protruding part that protrudes from the first or second surface of the layer, or is on the opposite site of the layer and connects to the one end of the protruding part that is inside the layer. The electrically conductive layer may be in electrically contact, and preferably in direct physically contact with the protruding part. The electrically conductive layer can be used to address the protruding part for signal transduction and/or stimulation purposes and/or penetration into the tissues for deep stimulation.

An electrode system according to the present disclosure comprises the above-described electrode element and further comprises at least a first connection portion (8) extending from the electrode element (B) in the x- and y-direction and/or in the xy-plane. The first connection portion (8) comprises a first base layer (8b) extending from the substrate base layer (1) of the electrode element (B) and being preferably integrally formed with the substrate base layer (1) of the electrode element (B). The first base layer (8b) is preferably based on and/or obtained by photopolymerization (curing) of the (same) mixture. The first base layer may be therefore formed and/or cured simultaneously with the substrate base layer. The first connection portion (8) comprises a further electrically conductive layer (8a) being deposited on the first base layer (8b) and being electrically and/or physically connected to the electrically conductive layer (5) of the electrode element (B). The further electrically conductive layer may serve as a feedline for supplying/applying currents/voltages to the electrically conductive layer (5) of the electrode element (B). The first base layer (8b) has a higher cross-linking density than the substrate base layer (1) of the electrode element (B), and the first base layer (8b) may have a higher stiffness than the substrate base layer (1). The first base layer (8b) has a larger thickness in the z-direction than the thickness (d₁) of the substrate base layer (1). The further conductive layer being deposited on the first base layer may be further connectable to an amplifier circuit or pulse generator for signal transduction or stimulation purposes if the substrate is used in a microelectrodes for bioelectronics that is brought into contact with tissue comprising electro-genic cells, e.g. nerve tissue.

A method for manufacturing any one of the above-mentioned substrate (A) according the present disclosure comprises disposing the mixture on a surface of a support (S), exposing the mixture with light, thereby the first surface (1-1) of the substrate base layer (1) is obtained on the side from which the light is irradiated, and the second surface (1-2) of the layer is obtained on the side opposite to the first surface in the z-direction. The light is preferably irradiated from a single direction.

Preferably, the method further comprises forming the band portion (2) by exposing only at a part of the mixture (10) and/or the substrate base layer (1) on the support (S) with light for a time length longer than a time length applied for the other part of the substrate base layer (1), and/or preferably, said part is exposed with light having an irradiance higher than an irradiance applied for the other part of the substrate base layer (1), so that the band portion (2) has a higher cross-linking density than the other part. The substrate base layer and/or at least the second surface of the substrate base layer is preferably at least partially or entirely covered by the mixture during the step of forming the band portion. Preferably, the method further comprises forming the dent portion (7) by irradiating a laser beam onto a part of the first surface (1-1) or onto the second surface (1-2) of the layer (1). Preferably, the method further comprises depositing the protruding part (3) or the embedded part on the surface of the support before the step of disposing of the mixture on the surface of the support (S), or depositing the protruding part (3) or the embedded part on the first surface (1-1) and/or the second surface (1-2) of the layer (1) after the step of exposing of the mixture with light, or forming the protruding part (3) by further exposing only a part of the substrate base layer (1) and/or the mixture (10) with light, so that the protruding part (3) protrudes from the first-surface (1-1). Preferably, the substrate base layer and/or at least the second surface of the substrate base layer is at least partially or entirely covered by the mixture during the step of forming the protruding part. Additionally or alternatively, forming the protruding part (3) by exposing only a part of the substrate base layer (1) with light in the mixture with light having irradiance higher than irradiance applied for the other part of the substrate base layer (1), so that the protruding part (3) protrudes from the first-surface (1-1). Preferably, the mixture is exposed with light through the support (S), so that the first surface (1-1) is obtained at an interface between the mixture and the support (S), or wherein the mixture is exposed with the light from a side of the mixture relative to the interface between the support (S) and the mixture so that the second surface (1-2) is obtained at the interface between the mixture and the support (S).

A method for manufacturing an electrode element (B) according to the present disclosure comprises manufacturing a substrate (A) according to one or more steps of the above-mentioned method. The method comprises depositing the electrically conductive layer (5, 6) on the surface of the support (S) before the step of disposing of the mixture, or depositing the electrically conductive layer (5, 6) on the substrate (A). Preferably, the method comprises depositing the further protruding part on the electrically conductive layer.

A method for manufacturing an electrode system (C) according to the present disclosure comprises manufacturing an electrode element (B) according to one or more steps of the above-mentioned method. The method further comprises further exposing the mixture (10) with light, thereby a first base layer (8b) is obtained, wherein a time length of exposing the mixture (10) with light at the position of the first base layer (8b) is longer than a time length of exposing the mixture (10) with light at the position of the substrate base layer (1) of the electrode element (B), and/or wherein irradiance of light exposing the mixture is higher at the position of the first base layer (8b) than irradiance of light exposing the mixture at the position of the substrate base layer (1) of the electrode element (B). The method further comprises depositing a further electrically conductive layer (8a) on the first base layer (8b).

The substrate base layer has preferably a thickness in a range of from 0.5 µm to 50 µm, a length of 90 µm to 10 mm, a width of 90 µm to 10 mm, the band portion has preferably a thickness in the y-direction in a range of 10 µm to 100 µm, the protrusion part protrudes from the first (or second) surface (and/or has a height of) preferably in a range of 10 to 100 µm, the protrusion part and/embedded part has preferably a diameter (a diameter of a cross-section of the protrusion or embedded part in the xy-plane) of 10 to 100 µm (50 µm), a dent portion has a diameter (in the xy-plane) of 10 to 100 µm (30 µm), the electrically conductive layer has preferably a thickness of 15 to 100 nm, (50 nm), the first base layer has preferably a thickness of 50 to 200 µm (150 µm), the further electrically conductive layer has preferably a thickness of 15 to 100 nm, (50 nm).

### BRIEF DESCRIPTION OF THE DRAWING

Figs. 1(a) and 1(b): schematic perspective and side views of a substrate according to the first embodiment
Figs. 2(a) and 2(b): schematic perspective and side views the substrate in a folded-state according to the first embodiment
Figs. 3(a) and 3(b): schematic explanations of manufacturing processes of the substrate according to the first embodiment
Figs. 4(a) to 4(c): pictures of a top view of the obtained substrate example according to the first embodiment
Figs. 5(a) and 5(b): pictures of contact angle measurements performed on the first and second surfaces of the substrate example according to the first embodiment
Fig. 6: a diagram which plots a curvature radius and a thickness of substrate examples against an exposure time length applied for curing the substrate examples according to the first embodiment
Figs. 7(a) and 7(b): schematic perspective and side views of a substrate according to the second embodiment
Figs. 8(a) and 8(b): schematic perspective and side views the substrate in a folded-state according to the first embodiment
Figs. 9 (a) to 9(c): pictures of a top view of a substrate example according to the second embodiment
Figs. 10 (a) to 10(c): pictures of a top view of a substrate example according to the second embodiment
Figs. 11(a) and 11(b): schematic side views of a substrate according to the third embodiment before and after folding
Figs. 12(a) to 12(c): schematic explanations of manufacturing processes of the substrate according to the third embodiment
Figs. 13(a) and 13(b): schematic side views of a substrate according to a variation of the third embodiment before and after folding
Figs. 14(a) to 14(d): schematic explanations of manufacturing processes of the substrate according to the variation of the third embodiment
Figs. 15(a) and 15(b): a schematic side view of a first electrode element and a second electrode element
Figs. 16(a) and 16(b): schematic explanations of manufacturing processes of the second electrode element
Figs. 17(a) and 17(b): schematic side views of a third and fourth electrode elements
Figs. 18(a) to 18(c): schematic explanations of manufacturing processes of the third and/or fourth electrode elements
Fig. 19: a schematic side view of an electrode element with one or more through-holes
Figs. 20(a) to 20(c): schematic top and side views of an electrode system

### DETAILED DESCRIPTION OF EMBODIMENTS

### 1. Substrate

### 1.1 First embodiment

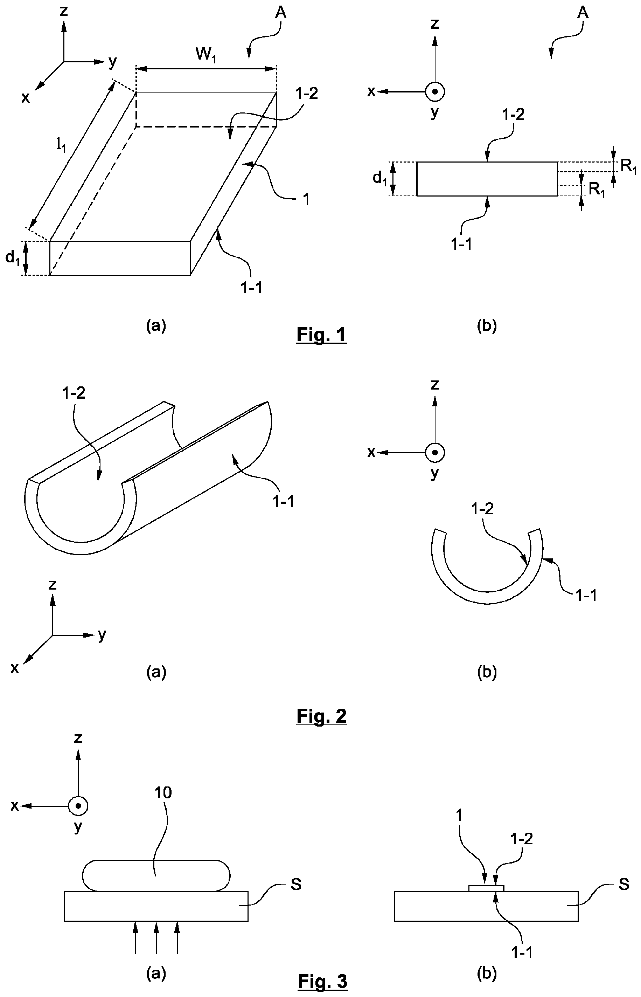

Figs. 1(a) and 1(b) schematically show a perspective and side view of the substrate (A) according to the first embodiment, respectively. The substrate (A) according to the first embodiment is a substrate base layer (1), which is a polymer compound layer having a length (l₁) in the x-direction, width (w₁) in the y-direction and a thickness (d₁) in the z-direction. The polymer compound layer (1) according to the first embodiment is obtained by curing a mixture (10) being a photocurable pre-polymer solution. The substrate (A) is self-foldable according to the hydrophilicity/hydrophobicity differences between the first and second surfaces (and/or the first and second sub-regions) of the polymer compound layer (1). The self-folding process is activated and/or induced by submerging the substrate (A) into liquid including water molecules.

The resin mixture for forming the substrate base layer (1) includes two main photocurable components; a hydrophobic component (first component (b)) and a hydrophilic component (second component (c)), each of which comprising at least oligomer or polymer or copolymer. The hydrophobic component may comprise, for example, silicone polymer having at least one first functional group. The hydrophilic component comprises, for example a hydrophilic monomer having at least one second functional group and is able to form a hydrogel by absorbing water molecules. The resin mixture further comprises at least one photoinitiator (a) for activating the first and second functional groups, so that the first and second components are polymerized through these functional groups.

The resin mixture may optionally further comprise one or more additional hydrophobic component (third component, d), which is for example a silicone polymer having a third functional group. The additional third component may contribute to control for example mechanical properties (such as elasticity and/or stiffness) of the polymer compound layer (1) and/or the polymerization process, for example, a speed of polymerization. The resin mixture may further comprise further components such as diluent, quenchers, photosensitizers. These additional components are only optional.

As illustrated in Fig. 3(a), the resin mixture (10) is at first casted on a glass support (S) and light (typically UV light in a range of 280 to 700 nm) is irradiated from a light source to the resin mixture (10) through the grass substrate (S) (in Fig. 3a, illustrated with arrows at a bottom part of the glass support (S)). The resin mixture (10) is preferably exposed with light from only a single direction and/or from only a single side, namely only in the z-direction in Fig. 3a, so that the mixture (10) is polymerized through the frontal photopolymerization process.

Upon irradiating light, the photoinitiator(s) (a) is at first activated by light and generates a radical, which reacts with and activates the functional groups of the hydrophobic component (b) and the hydrophilic component (c) to form a polymer network. A polymerized front surface is at first formed closest to the light source (i.e. at the interface between the resin mixture (10) and the glass support (S)). The polymerization inside of the resin mixture proceeds gradually in the z-direction, and the front surface, i.e. the interface between the uncured resin mixture and the curing resin mixture, moves inside the uncured mixture in the z-direction, exclusively or mostly as long as the light is irradiated. Here, the curing resin mixture part refers to a part between the polymerized front surface and the interface of the mixture (10) and the support (S), and inside of the curing resin mixture polymerization through the first and/or second functional groups is undergoing. The cross-linking density of the curing resin mixture part further increases as long as the light is irradiated, namely, its (average) polymer mesh size further decreases, and its polymer density further increases during the curing.

After the light exposure step, uncured residues of the resin mixture are removed e.g. by rinsing with organic solvent, and the substrate base layer (1) is obtained on the glass support (S) (cf. Fig. 3(b)). The substrate base layer (1) may be removed from the glass support manually or by rinsing with water. The polymer compound layer (1) has the first surface (1-1), which has been produced on the side from which the light is irradiated (i.e. at the interface of the resin mixture and the substrate (S)). The substrate base layer (1) has typically an inhomogeneous cross-linking density along the z-direction, namely the substrate base layer (1) has a gradient of conversion along the z-direction (i.e. along the direction into which light is irradiated), as the cross-linking density has a correlation with deposited light doses, which decreases exponentially along the z-direction inside of the curing resin mixture. The cross-linking density may be therefore highest at the first surface (1-1) and/or the first sub-region (R₁).

The substrate base layer may be further inhomogeneous along the z-direction in terms of the number of the hydrophobic and hydrophilic components, due to the difference of the molecular weight and/or diffusivity of the components. More specifically, the number of the hydrophobic component (or the hydrophilic component) changes or differs along the z-direction of the substrate base layer, and the layer accordingly exhibits a distribution gradient of the hydrophobic component (or the hydrophilic component) along the layer thickness: During the photopolymerization process, a phase separation would occur inside the curing resin mixture in the z-direction. The hydrophilic component having a smaller molecular weight than the hydrophobic component is likely polymerized faster than the hydrophobic component, as the hydrophilic component may have a high diffusivity or mobility in the curing resin mixture. The hydrophobic components may tend to be polymerized in a region closer to the light source, i.e. at a region where the energy of attenuated light is higher, and the more of the hydrophilic components may tend to be polymerized in a region away from the light source. The substrate base layer (1) would accordingly become inhomogeneous in terms of hydrophilicity and/or hydrophobicity along the z-direction through the frontal photopolymerization process, and the first surface (1-1) is more hydrophilic than the second surface (1-2). The second surface (1-2) is accordingly more hydrophobic than the first surface (1-1). The polymer compound layer (1) may accordingly include a first sub-region (R₁) including the first surface (1-1) and having a first thickness from the first surface (1-1) in the z-direction, and the first sub-region (R1) would be richer in the hydrophilic components. More specifically, the first sub-region (R₁) may contain larger numbers of the hydrophilic components (a) than the hydrophobic components (b). At the opposite side of the substrate (A) in the z-direction, a second sub-region (R₂) including the second surface (1-2) and having a second thickness from the second surface (1-2) in the z-direction may be formed. The layer (1) may include an intermediate region between the first and second sub-regions. The second sub-region (R₂) would contain more numbers of the hydrophobic components (b) than the hydrophilic components (a). The substrate base layer (1) may accordingly have a concentration gradient and/or inhomogeneous concentrations of the hydrophilic components (a) (and/or the hydrophobic components (b)) along the z-direction, i.e. the thickness of the polymer compound layer (1).

The unique characteristics of the substrate base layer (1) allows a self-assembling process, which is triggered by liquid. More specifically, the substrate (A) is self-foldable according to the hydrophilicity/hydrophobicity differences between the first and second surfaces (and/or the first and second sub-regions). The self-folding process is activated and/or induced by submerging the substrate base layer (1) into liquid including water molecules or by bringing at least the first surface (1-1) and/or the first sub-region (R₁) into contact with liquid including water molecules. The first surface (1-1) (and/or the first sub-region (R₁)) being more hydrophilic absorbs more water molecules than the second surface (1-2) (and/or the second sub-region (R₂)), which results in a higher (planar and/or volumetric) expansion of the first surface (1-1) (and/or the first sub-region (R₁)) than the second surface (1-2) (and/or the second sub-region (R₂)). The high expansion of the first surface and/or sub-region (R₁) creates a stress against the second surface and/or sub-region (R₂). The stress is released by self-folding of the substrate base layer (1), and the substrate base layer (1) is folded such that a side of the second surface (1-2) is concave and a side of the first surface (1-1), as illustrated in Figs. 2(a) and 2(b). More specifically, the folded substrate base layer (1) may have a cylinder-like shape in which the second surface (1-2) is inside and the first surface (1-1) is outside of the folded layer.

The cross-linking density may further have correlation with mechanical properties of the cured polymer, and the higher cross-linking density may lead to lower elasticity (higher stiffness), i.e. lower mechanical flexibility. The substrate base layer (1) may therefore additionally have a gradient of elasticity (which may be determined for example by young modulus) along the z-direction.

Preferably, the photopolymerization and/or frontal photopolymerization of the resin mixture is performed by irradiance in a range of 3.1×10⁻¹ to 6.2 mW cm⁻², more preferably 3.1×10⁻¹ to 6.2 × 10⁻¹ mW cm⁻², for the exposure time in a range of 5 to 30 sec, more preferably 8 to 15 sec, in a room temperature. The volume of the resin mixture is preferably in a range of 0.1 µl/mm² to 10 µl/mm², more preferably in a range of 0.2 µl/mm² to 0.4 µl/mm². A curvature radius of the folded substrate base layer (1) (i.e. the folded substrate), which is defined by the second surface (1-2) being an internal surface of the folded substrate base layer, is preferably in a range of a targeted biological sample to be wrapped by the folded substrate base layer (1), such as a nerve fiber, and for example in a range of 100 µm to 1 cm, more preferably 100 to 500 µm.

The first component may be one of DBE-U22, UMS-992, DMS-U21, UTT-1012, SIA0200, DMS-V05, DMS-V21, DMS-V22, DMS-V25, UMS-182, UCS-052, and the second component may be one of DMA, PEGDA (Mₙ=250), PEGDA (Mₙ=500), PEGDA (Mₙ=700), HEMA. According to experimental results (not shown) of Inventors, more preferably, the first component may be selected from DBE-U22, UMS-992, DMS-U21, UTT-1012, SIA0200, and the second component may be selected from DMA, HEMA, PEGDA (Mₙ=250), which would be advantageous to obtain the resin mixture being well mixed and homogeneous mixture. Preferably, when the first component is and/or comprises UMS-992, the second component is and/or comprises HEMA and/or DMA and/or PEGDA (Mₙ=250) and/or PEGDA (Mₙ=500) and/or PEGDA (Mₙ=700). In particular, PEGDA (Mₙ=500) and/or PEGDA (Mₙ=700) may be advantageous to obtain a high volumetric expansion when absorbing water molecules. According to experimental results (not shown) of Inventors, the resin mixture comprising DBE-U22 as being the first component may be especially advantageous to obtain a small curvature radius of a folded substrate base layer in a range of 100 µm to 500 µm. According to further experimental results (not shown) of Inventors, the resin mixture comprising DMA as being or being comprised in the second component may be advantageous to obtain a small curvature radius of a folded substrate base layer. The above mentioned components are only suitable examples and the first and second components are not limited thereto.

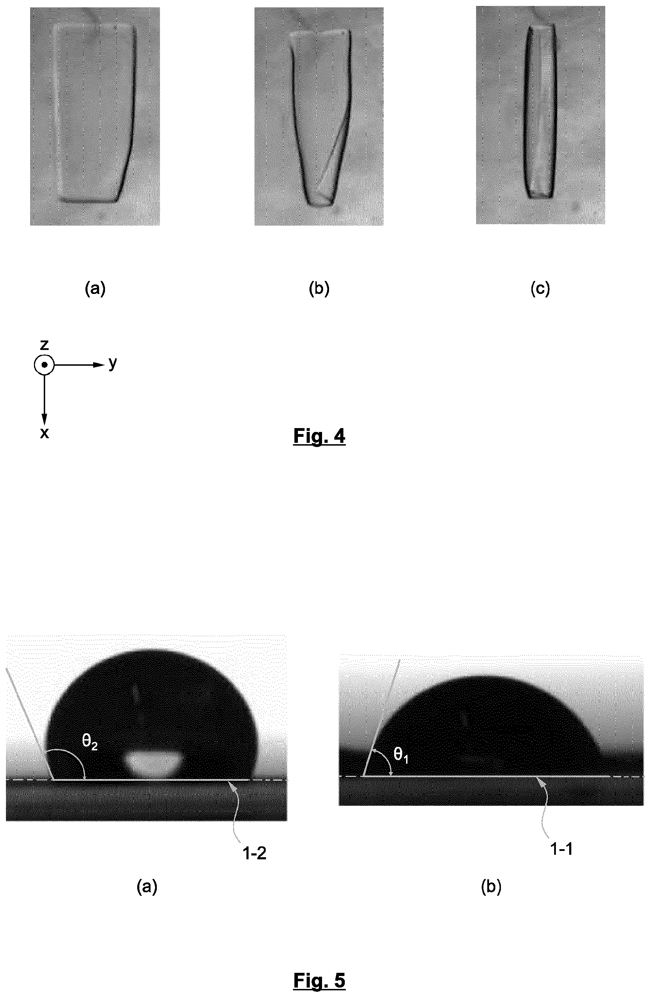

The first embodiment is explained by substrate examples shown in Figs. 4(a) to 6. The components and conditions used for the substrate examples are only example and do not restrict the first embodiment.

### 1.1.1 Example 1

The resin mixture was prepared by mixing silicone resin (DBE-U22 and UMS-182, each of which is an example of the first component and/or the third component), the hydrogel monomer N,N-dimethylacrylamide (DMA) (which is an example of the second component), and amphiphilic acrylic acid isobornyl ester (IBA, which is diluent) and bis(2,4,6-trimethylbenzoyl) phenylphosphine oxide/ethyl(2,4,6-trimethylbenzoyl) phenylphosphinate (which is an example of a photo initiator), 2-isopropylthioxanthone (2-ITX), and 2,2,6,6-tetramethylpiperidine-1-oxyl (TEMPO) (each of which is an example for free-radical quencher, and UV absorber, respectively). The volumetric ratio of the silicone resin to the hydrogel was 1:3. The resin mixture was stirred for 30 min, stored at 4°C, and brought back to room temperature before usage. The resin mixture (e.g. 200 µL) was casted on a glass support (24 mm×24 mm, 120 µm thickness) and was exposed to light for curing into a rectangular pattern 1.2 mm×2.4 mm. A digital light processing 3D printer (Ultra50, MiiCraft) with a wavelength of 365 nm, maximum irradiance of 6.2 mW cm⁻², and pixel size of 30 µm was used as a light source. The resin mixture was exposed to the light intensity with irradiance ranging from 3.1 ×10⁻¹ mWcm⁻² for 13 sec. Subsequently, uncured residues have been removed by rinsing with isopropanol. The substrate example had a layer thickness (d₁) of 15 µm.

Fig. 4(a) shows a picture of a top view (i.e. a view from the second surface (1-2)) of the obtained substrate example according to the first embodiment. The substrate example was subsequently submerged into deionized water, so that the substrate example (or at least the first surface and/or the first sub-region of the substrate example) were swelled and saturated by deionized water. Fig. 4(b) shows a picture of the substrate example at 0.3 sec after having been submerged into deionized water. The flat-patterned substrate example started self-folding and then finally formed a cylindrical folded-state (cf. Fig. 4(c) showing a picture of the substrate example at 1 sec after having been submerged into deionized water), such that the second surface (1-2) is an inner surface and the first surface (1-1) is an outer surface of the folded substrate example. The substrate example folded in the y-direction, namely the folded axis was defined in the x-direction, into which the substrate example has a longer side (corresponding to the length l₁ in Fig. 1a).

Figs. 5(a) and 5(b) show contact angle measurements performed on the second surface (1-2) and first surface (1-1), respectively, of the substrate example according to the first embodiment. The second surface (1-2) shows the contact angle of θ₂~ 100°, whereas the first surface shows a contact angle of θ₁ ~ 83°. This result shows that the first surface (1-1) has a higher hydrophilicity than the second surface (1-2).

### 1.1.2 Examples 2

Further substrate examples #1 to #10 were prepared based on the resin mixture with hydrophobic polymer (DBE-U22, which is an example of the first component) and hydrophilic monomer (DMA, which is an example of the second component). In these examples, the mixture did not include the third component such as UMS-182 used in Example 1. Other components of the resin mixture were identical to those of Example 1. The substrate examples #1 to #10 (each of which being a rectangular pattern 1.2 mm×2.4 mm) were obtained by curing the resin mixture with light (irradiance 3.1 × 10⁻¹ mW cm⁻²) for various exposure time lengths, in a range from 7 sec. to 24 sec. The substrate examples #1 to #10 were submerged into and saturated by deionized water, and a curvature radius of each example in the folded-state has been measured. The curvature radius was defined by the internal surface (i.e. the second surface) of the folded substrate examples.

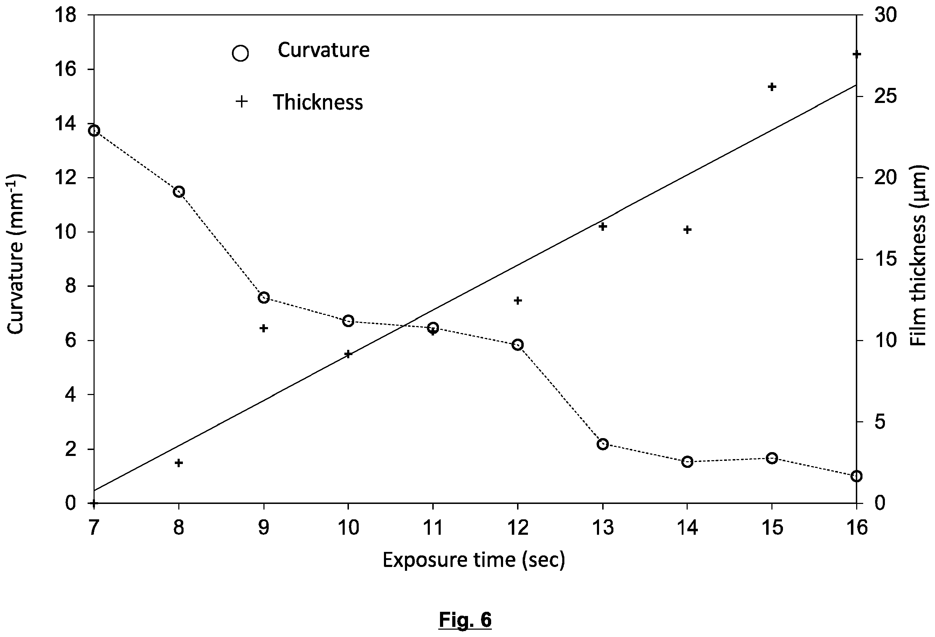

Fig. 6 shows a diagram which plots a curvature radius (mm⁻¹, left vertical axis, circle markers) and a thickness (µm, right vertical axis, cross markers) of the substrate examples #1 to #10 against an exposure time length applied for curing the substrate examples #1 to #10. The layer thickness (d₁) linearly increases with the exposure time length and varies in a range between 1 to 28 µm. The curvature varies in a range between 1 to 14 mm⁻¹ (which corresponds to 900 to 70 µm in curvature radii), and the results show that a lager curvature radius is obtained for a larger layer thickness, i.e. a longer exposure time. The curvature radius of the folded substrate can be thus precisely controlled by tuning the substrate base layer thickness. Furthermore, the results show that each of the substrate examples yields a curvature radius in a range of several hundred micrometers, which may be particularly suitable for wrapping a biological sample such as a nerve fibre. The substrate according to the first embodiment is especially suitable for a cuff-electrode application.

### 1.2 Second embodiment

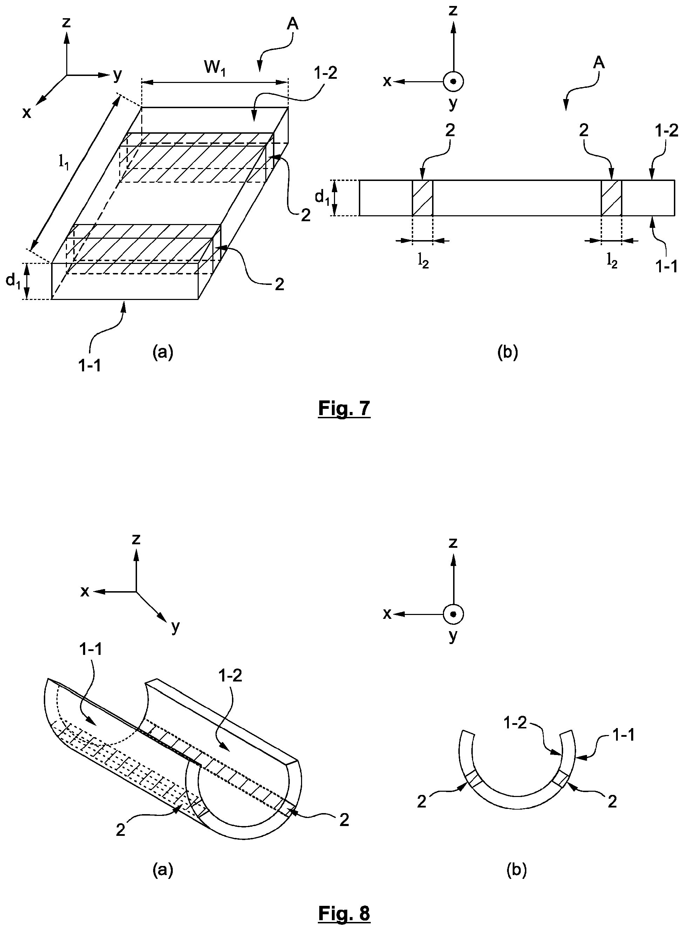

Figs. 7(a) and 7(b) schematically show a perspective and side view of the substrate (A) according to the second embodiment, respectively. The substrate (A) according to the second embodiment is a substrate base layer (1) being a polymer compound layer, which further comprises at least one band portion (2) being formed inside of the substrate base layer (1). The band portion (2) is formed by further exposing a part of the substrate base layer (1) according to the first embodiment with light, so that this exposed part is further polymerized by cross-linking the first and/or second components. The band portion (2) is therefore a part of the substrate base layer being a polymer compound layer and consists of the same resin mixture of the (other part of) polymer compound layer (1). More specifically, the resin mixture is cured by the (frontal) photopolymerization process to obtain the substrate base layer (1) having a length (l₁), width (w₁) and thickness (d₁) as described for the first embodiment. Subsequent to or simultaneous to this (frontal) photopolymerization, only a part(s) of the substrate base layer (1) (hereinafter the second curing step) is further polymerized by irradiating this part with light for a longer time and/or by irradiating this part with a higher irradiance of light than the other part of the substrate base layer (1). The band portion (2) has accordingly a higher cross-linking density than the other part of the substrate base layer (1) and/or at least the second surface (1-2) and/or the second sub-region (R₂).

The band portion (2) has a longer side elongating in the y-direction and has a smaller side being a thin thickness l₂ in the x-direction, such that the band portion (2) internally divides or separates at least a part of the substrate base layer (1) into at least two regions in the x-direction. The longer side of the band portion (2) may be parallel to the y-direction or form an angle with the y-direction and/or parallel to the x-direction. The band portion (2) may have a height in the z-direction being same to the thickness d₁ of the substrate base layer (1). Alternatively, the height of the band portion (2) may be larger than the thickness d₁ of the substrate base layer (1), and a part of the band portion (2) may protrude from the second surface (1-2) of the substrate base layer (1).

The band portion (2) may be especially advantageous for more accurately controlling the folding direction of the substrate (A) and/or the substrate base layer (1). When at least the first surface (1-1) and/or the first sub-region (R₁) (other than the band portion (2)) of the substrate base layer (1) is swelled by liquid components, the substrate base layer (1) folds by itself, such that the second surface (1-2) is the inner surface of the folded substrate base layer). In this folding process, the band portion (2) having a higher rigidity may restrict the folding of the layer (1) in the y-direction, which is the main longitudinal direction (i.e. longer side) of the band portion (2), and the substrate base layer (1) accordingly folds up in the x-direction, i.e. in the direction (substantially) orthogonal to the main longitudinal direction of the band portion (2) (cf. Fig. 8a). More specifically, the folded layer would have a folding-axis along the y-direction and the curvature radius of the folded substrate would be defined in the xz-plane (cf. Fig. 8b). When a plurality of the band portions is formed, the curvature radius of the folded layer may be controlled by a number of the band portions (2) and/or a distance between each neighboring band portion. The greater number of the band portions (2) and/or smaller distance between each neighboring band portion may increase (average) stiffness of the substrate base layer (1) and the curvature radius of the folded layer would become larger.

To better control the folding direction, the band portion (2) preferably extends for 80 % to 100 % of the of width (w₁) of the substrate base layer (1) in the y-direction. The band portion (2) has preferably the thickness (l₂) in the x-direction less than 10 %, more preferably less than 5 % of the length (l₁) of the substrate base layer, so that the substrate remains flexible to allow the self-folding process.

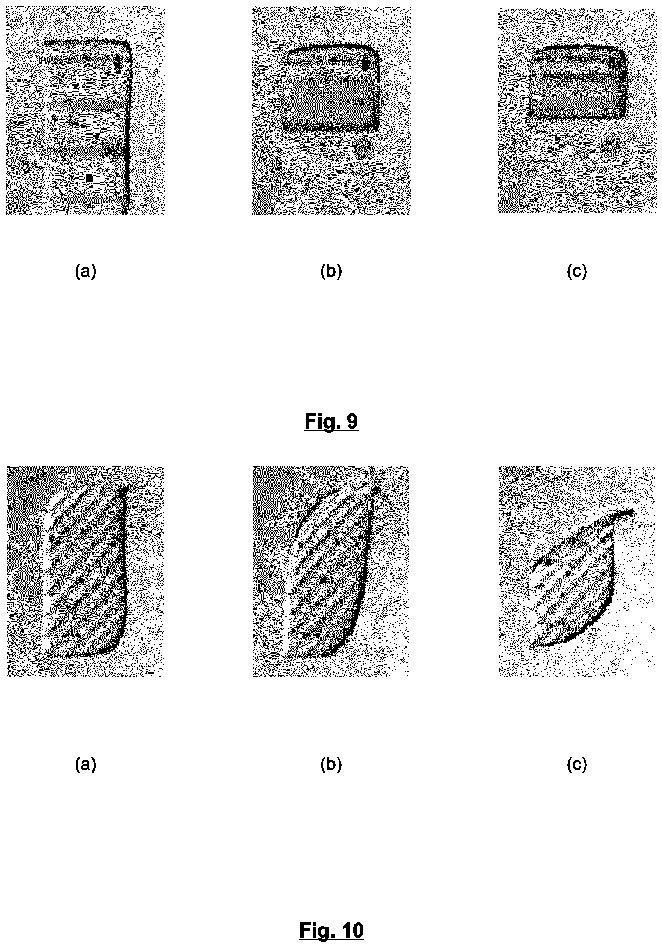

The second embodiment is explained by substrate examples shown in Figs. 9 and 10. The components and conditions used for the substrate examples are only example and do not restrict the second embodiment.

### 1.2.1 Examples 3 and 4

Figs. 9 and 10 show a picture of a top view (i.e. a view from the second surface (1-2)) of the substrate examples according to the second embodiment. The same resin mixture used for Example 1 was cured by the frontal photopolymerization process as described in Example 1, so that the substrate base layer (1) according to Example 1 is at first cured. A plurality of the band portions (2) was subsequently formed by a local exposure of light at irradiance 9.3×10⁻¹ mW cm⁻², with exposure time 10 sec. in a state in which the cured substrate base layer (1) is entirely covered by the resin mixture (i.e. pre-polymerized solution). The unreacted residue was subsequently removed by rinsing the substrate examples with isopropanol.

Fig. 9(a) shows a picture taken from a top (i.e. the second surface (1-2)) of a substrate example according to Example 3. The band portions (2) being visible in the picture as darker stripes are parallel to the y-direction and separated from each other in the x-direction with a distance of 210 µm. Fig. 9(b) shows a picture of the substrate example at 0.3 sec after having been submerged into deionized water. The flat-patterned substrate example started self-folding in the x-direction and then finally formed a cylindrical folded-state (cf. Fig. 9(c) at 1 sec after having been submerged into deionized water), such that the second surface (1-2) is an inner surface and the first surface (1-1) is an outer surface of the folded substrate example. The substrate example thus folded in a direction being substantially orthogonal to the longer side of the band portions.

Fig. 10(a) shows a picture taken from a top (i.e. the second surface (1-2)) of a substrate example according to Example 4. The band portions (2) are visible in the picture as darker stripes and each of the band portions (2) forms an angle, ~ 45°, with the y-direction. Each band portion (2) being visible in the picture as darker stripes forms an angle, ~ 45°, with the y-direction. The band portions are separated from each other with a distance of 60 µm. Fig. 10(b) shows a picture of the substrate example at 0.3 sec after having been submerged into deionized water. The flat-patterned substrate example started self-folding in a direction being diagonal to the x- and y-directions, then finally formed a cylindrical folded-state (cf. Fig. 10(c) at 1 sec after having been submerged into deionized water), such that the second surface (1-2) is an inner surface and the first surface (1-1) is an outer surface of the folded substrate example. The substrate examples folds in a direction being substantially orthogonal to the longer side of the band portions.

### 1.3 Third embodiment

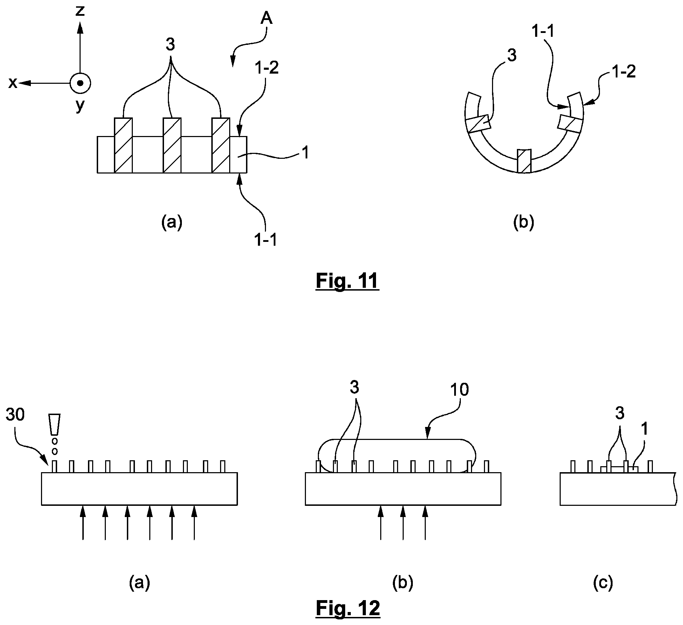

Fig. 11(a) schematically shows a side view of the substrate (A) according to the third embodiment. The substrate (A) according to the third embodiment comprises the substrate base layer (1) according to the first and/or second embodiment and further comprises at least one protruding part (3) protruding from the second surface (1-2) of the substrate base layer (1) in the z-direction. The protruding part (3) may extend through the polymer layer (1) in the z-direction. When the substrate (A) is submerged into liquid including water molecules, the substrate (A) (i.e. the substrate base layer (1)) folds itself, such that the second surface (1-2) is an inner surface of the folded substrate (A). The protruding part (3) accordingly would be disposed inside of the folded substrate (A) and project into the inside of the folded substrate (A) (cf. Fig. 10(b)). The substrate (A) according to the third embodiment may be especially advantageous in that the protruding part (3) can more stably mechanically fix a biological sample being wrapped by the folded substrate. The protruding part (3) may further serve as a part of an electrode for applying currents/voltages to the target biological sample.

The substrate (A) according to the third embodiment may be manufactured by adding a step of forming the protruding part (3) to the manufacturing steps according to the first and/or second embodiment. As illustrated in Fig. 12(a), a photocurable resin mixture is deposited (directly or on a sacrificial layer) on the glass support (S) by an inkjet printer and subsequently cured by irradiating light into a pillar and/or rod shape, so that one or more protruding parts (3) are deposited on the glass support (S). The protruding part (3) may be obtained by curing a photocurable resin being different from or same to the resin mixture for the substrate base layer (1). Alternatively, the protruding part (3) may be a conductive material, formed by for example Ag nanoparticle embedded photocurable resin mixture. The protruding part (3) may be a particle, such as a microbead ink and/or a droplet, and/or pillars and wires. Subsequently, the resin mixture for the substrate base layer (1) is casted on the glass support (S), where the protruding part (3) are deposited. The resin mixture is further cured by irradiating light through the glass support (S) via the frontal photopolymerization process, as already explained in the first embodiment (cf. Fig. 12b). The thickness (d₁) of the polymer compound layer (1) is controlled by the light exposure time length of the first curing step or by a volume of the resin mixture, such that at least a part of the protruding part (3) protrudes from the second surface (1-2) of the polymer compound layer (1) (cf. Fig. 12c). The band portion (2) as explained in the second embodiment may be additionally formed in the substrate base layer (1) with the protruding part (3).

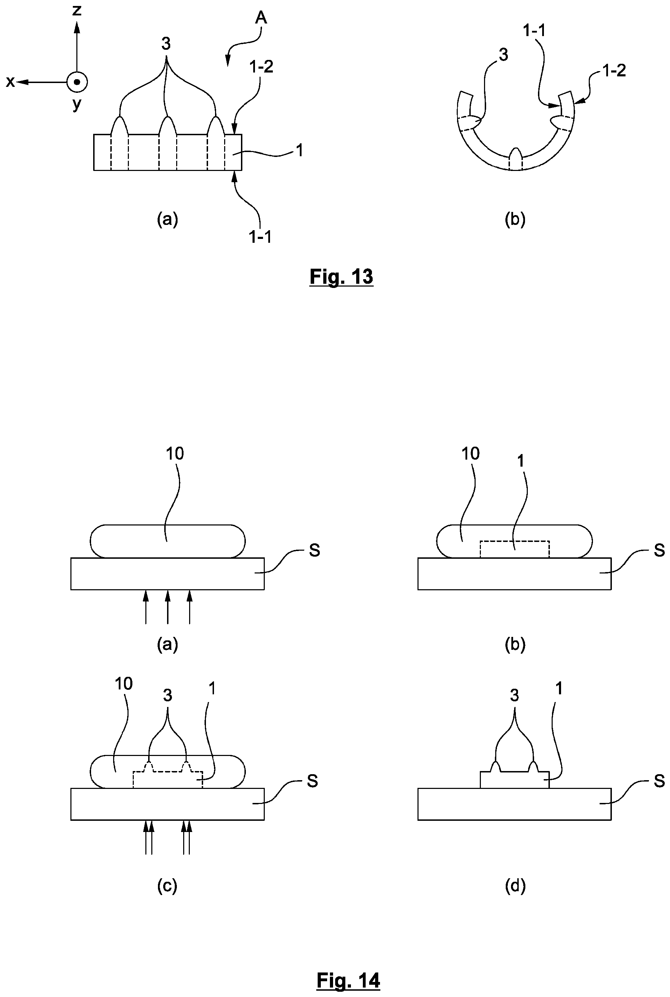

Figs. 13(a) schematically shows a side view of a substrate (A) according to a variation of the third embodiment. The protruding part (3) may be integrally formed with the substrate base layer (1). More specifically, the protruding part (3) may be obtained by photopolymerization of the same mixture for the substrate base layer (1). The protruding part (3) is formed by further exposing only a part of the substrate base layer (1) according to the first/second embodiment with light in the resin mixture, so that this exposed part is further polymerized and grows in the z-direction from the second surface (1-2) of the substrate base layer (1). As illustrated in Figs. 14(a) and 14(b), the resin mixture (10) is casted on the glass substrate (S) and is cured by irradiating light through the substrate (S) to obtain the substrate base layer (1) as described in the first and/or second embodiment. Subsequent to or simultaneous to this (frontal) photopolymerization process, only a part of the substrate base layer (1) (for example an area with a diameter of 50 µm) is further polymerized by irradiating this part with light for a longer time and/or by irradiating this part with a higher irradiance of light than the other part of the substrate base layer (1) (cf. Figs. 14(c) and 14(d)). The protruding part (3) may therefore have a higher cross-linking density and/or higher stiffness than the other part of the substrate base layer (1). When the substrate (A) is submerged into liquid, the substrate (A) folds such that the protruding part (3) protrudes into the inside of the folded substrate (cf. Fig. 13(b)). The protruding part (3) may have a height (i.e. a length from the plane of the second surface (1-2) to an edge of the protruding part (3)) in a range of 10 to 50 µm, more preferably 30 to 40 µm. The protruding part (3) according to the modification of the third embodiment may have better mechanical stability as the protruding part (3) is integrally formed with the substrate base layer (1).

The third embodiment is explained by Examples 5 and 6. The components and conditions used for the substrate examples are only example and do not restrict the third embodiment.

### 1.3.1 Example 5

The substrate example according to the third embodiment was manufactured as following: A sacrificial layer (a gelation layer of Ca-alginate formed by sodium alginate and calcium chloride) was coated on the glass support (S). A photocurable epoxy ink (DM-IN-7010S, Dycotec) was loaded into a disposable 1 pico-liter cartridge, and the ink was printed on the sacrificial layer with an inkjet printer. The individual ink droplet has a diameter 60 µm to grow (pre-cured) pillar-shaped arrays. The printed ink was then cured by UV light, so that a plurality of the protruding parts is formed on the sacrificial layer. The obtained protruding parts had a height ranged from 10 to 2000 µm in the z-direction. Subsequently, the resin mixture being used for Example 1 and 2 was casted on the sacrificial layer where the protruding parts (4) are deposited, so that the resin mixture covers the protruding parts. The same steps of the Example 1 and 2 were carried out to obtain the substrate base layer (1) having a thickness d₁ of 10 µm.

### 1.3.2 Example 6

The resin mixture being used for Example 1 was casted on a glass substrate (S) and the same steps of the Example 1 were carried out to obtain the substrate base layer (1). A part of the obtained substrate base layer (1) was subsequently exposed with light in a state in which the substrate base layer is entirely covered by the mixture (i.e. pre-polymer solution) for forming the protruding part (3) as depicted in Fig. 13. The irradiated UV light has a spot size having a diameter of 30 µm, with irradiance of 9.3×10⁻¹ mW cm⁻² for 15 sec. The obtained substrate (A) had the substrate base layer (1) having a thickness d₁ of 15 µm and the protruding part having a height of 30 µm.

### 1.3.3 Further modifications of the third embodiment

The substrate (A) may comprise at least one embedded part being embedded in the substrate base layer (1) according to the first and/or second embodiment. Similar to the third embodiment, the embedded part preferably has a rod-shape or a pillar shape being elongated in the z-direction, and the embedded part may be obtained by the same manufacturing process as described in the third embodiment, in which the height of the embedded part and the thickness d₁ of the substrate base layer (1) are controlled such that ends of the embedded part do not protrudes from the first/second surfaces of the substrate base layer (1). The first and/or second ends of the embedded part are exposed to the outside of the layer (1) and are physically contactable from the outside of the layer (1). The second end of the embedded part is preferably disposed within or between the first and second surface (1-2) of the layer (1).

The embedded part may extend through the polymer compound layer (1), such that the first and second ends of the embedded part are exposed to the outside of the layer. Alternatively, only one of the first and second ends of the embedded part may be exposed to the outside of the layer. For example, the second end of the embedded part may be disposed on the side of the second surface (1-2) and the first end of the embedded part may be entirely embedded inside of the layer (1). Alternatively, the first end of the embedded part may be disposed on the side of the first surface (1-1) and the second end of the embedded part may be entirely embedded inside of the layer (1).

### 1.4 Further modifications

The substrate base layer (1) of the substrate (A) according to any one of the above-described embodiments may further comprise a dent portion and/or a through hole extending in the z-direction. For example, one or more through-holes may be opened in the z-direction, preferably besides the band portion (2) and/or the protruding portion (3). The through-hole has preferably a diameter (i.e. a diameter of a cross-section of the through-hole in the xy-plane) of 30 to 120 µm. The through-hole may serve for example as an access passage of e.g. oxygen, nutrients, or chemical stimulants for a biological sample to be wrapped by the substrate (A).

### 2. Electrode Element

The substrate (A) according to any one of the above-described embodiments may be integrated in an electrode element (B), which comprises an electrically conductive layer (hereinafter conductive layer) deposited on the substrate (A). The electrode element (B) is for being implanted into a biological sample and would serve as an electrode site for directly contacting the biological sample. The conductive layer may be deposited on (i) the second surface (1-2) or (ii) the first surface (1-2) of the polymer compound layer (1), as explained below.

### 2.1 First and second electrode elements

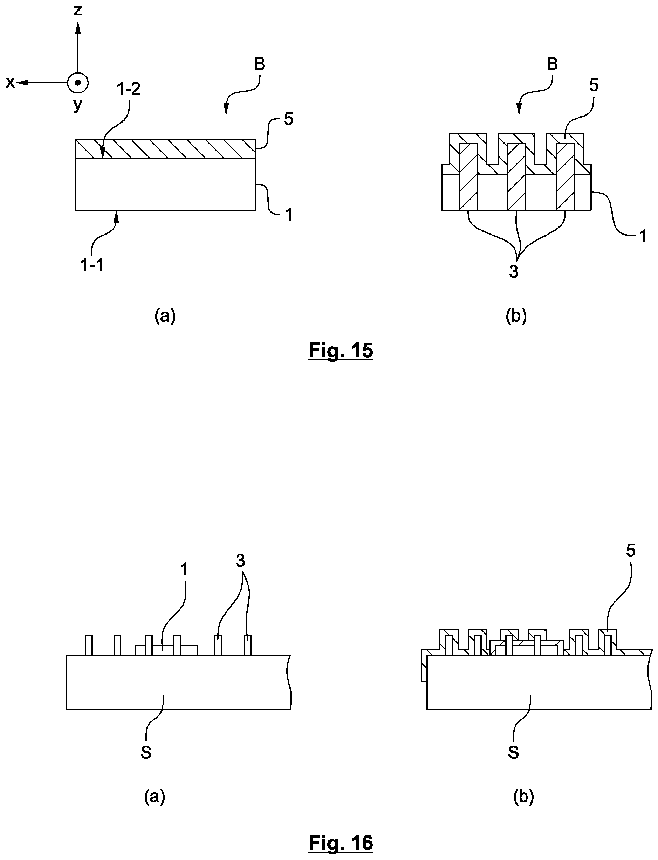

Fig. 15(a) schematically shows a side view of a first electrode element (B) according to an embodiment. The first electrode element (B) includes the substrate (A) according to the first or second embodiment. A conductive layer (5) is deposited on (the side of) the second layer (1-2) of the substrate base layer (1). The first surface (1-1), the second surface (1-2) and the conductive layer (5) are arranged and overlap each other in the z-direction in this order. The conductive layer (5) is deposited for example by sputtering a metal (e.g. gold), preferably for a thickness of 5 to 50 nm, more preferably 10 to 15 nm, more preferably in the z-direction. When the first electrode element (B) is submerged into liquid, the first electrode element (B) folds itself such that the conductive layer (5) is an inner surface of the folded electrode element (B), because of the folding mechanism of the substrate base layer (1).

Fig. 15(b) schematically shows a side view of a second electrode element (B) according to an embodiment. The second electrode element includes the substrate according to the third embodiment, which includes at least one protruding part (3). A conductive layer (5) is deposited on the substrate (A) of the third embodiment, such that the conductive layer (5) covers a surface of the protruding part (3) and the second surface (1-2) of the polymer compound layer (1-2). Alternatively, the second electrode element (B) may be formed by depositing the conductive layer (5) on the substrate (A) according to the variation of the third embodiment shown in Fig. 13(a), in which the protruding part (3) is integrally formed with the substrate base layer (1). Alternatively, a further protruding part may be deposited on the conductive layer (5). The protruding conductive layer (i.e. the conductive layer (5) being deposited on the protruding part (4)) can further serve as a part of an electrode applying and/or measuring electrical signals to/from a biological sample. The second electrode element (B) may be especially advantageous in that the protruding conductive layer may more efficiently interact with the biological sample, as the protruding part (3) would promote pressing the conductive layer (5) onto the biological sample. The protruding conductive layer may further contribute more stably physically attaching/fixing the electrode element to the biological sample, thereby yielding a stable and more reliable measurement. The thickness of the conductive layer (5) is preferably smaller than the height of the protruding part (3) protruding from the second surface (1-2) in the z-direction. More specifically, a surface of the conductive layer (5) is higher at the part of the protruding part (3) than the other part of the conductive layer (5) in the z-direction, so that a projected pattern is formed on the side of the second surface (1-2) (cf. Figs. 15(b)).

The second electrode element (B) may be formed by performing a step of depositing the conductive layer (5) subsequent to all steps for manufacturing the substrate (A) according to the first or second or third or fourth embodiment: The substrate (A) according to the first or second or third or fourth embodiment is at first prepared on the glass support (S), and subsequently, the electrical conductive layer (5) is deposited on the second surface (1-2) and/or the protruding part (3). A gold layer may be deposited on the substrate (A) as the conductive layer (5) by a sputtering process.

### 2.2 Third and fourth electrode elements

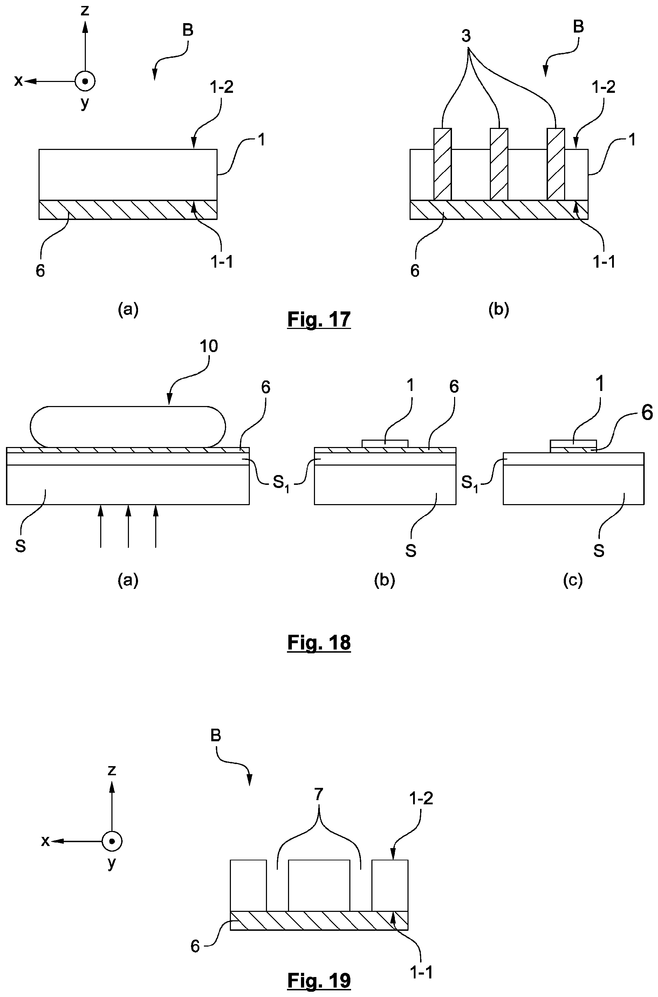

A third and fourth electrode elements according to embodiments, each of which comprises a conductive layer (6) deposited on (the side of) the first surface (1-1) of the substrate base layer, is schematically shown in Figs. 17(a) and 17(b), respectively. In each of the third and fourth electrode element, the conductive layer (6), the first surface (1-1) and the second surface (1-2) are stacked and overlap each other in the z-direction in this order. When the third electrode element (B) is submerged into liquid, the electrode element (B) folds itself by the folding mechanism induced by the substrate (A)/substrate base layer (1), such that the conductive layer (6) is an outside surface and the second surface (1-2) of the polymer compound layer (1) is an inner surface of the folded electrode element (B).

For manufacturing the third electrode element (B), the glass support (S) is at first covered by a sacrificial layer (S1) (for example gelatin or calcium hydrogel) and subsequently the conductive layer (6) is deposited on the sacrificial layer (S1) by for example chemical vapor deposition (CVD), spraying, thermal deposition, or transferring the CVD-grown materials, preferably for a thickness of 5 nm to 5 µm in the z-direction. The conductive layer (6) is preferably optically transparent at least for a range of light wavelengths used for curing the resin mixture, so that light can pass through the glass substrate and the conductive layer (6). The conductive layer (6) may be for example a monolayer or multilayer of graphene or indium tin oxide (ITO). The substrate (A) is subsequently deposited on the conductive layer (6), as described in any one of the above mentioned embodiment: The resin mixture (10) is casted on the electrical conductive layer (6) and is exposed to light through the glass substrate (S) and the conductive layer (6), so that the first surface (1-1) of the polymer compound layer (1) is formed at the interface of the electrical conductive layer (6) with the resin mixture (10) (cf. Fig. 18(a)). Subsequently, for example, oxygen plasma may be applied for removing residues of the conductive layer (cf. Figs. 18(b) and 18 (c)). The electrode element (B) may be manually detached from the glass support.

Fig. 17(b) schematically illustrates a side view of a fourth electrode element (B) comprising at least one protruding part (3), which protrudes from the second surface (1-2) of the substrate base layer (1) in the z-direction. In addition to the manufacturing steps described for the third electrode element (B), a step for depositing the protruding part (3) on the surface of the conductive layer (6) is performed prior to the step of casting the resin mixture (10) on the conductive layer (6). The step for depositing the protruding part (3) is identical to that of the third embodiment of the substrate (A). The protruding part (3) is preferably electrically conductive and is electrically and/or physically connected to the conductive layer (6), so that currents/voltages may be applied/measured through the protruding part (3) and the conductive layer (6).

As illustrated in Fig. 19, the third and/or fourth electrode element (B) may further comprise one or more through-holes (7) extending through the polymer compound layer (1) in the z-direction. The through-hole (7) may be opened by irradiating a laser beam onto the second surface (1-2) of the polymer compound layer (1).

### 3. Electrode system

Any one of the above-described electrode elements (B) may be integrated into an electrode system (C), which further comprises a feed line and a contact pad for supplying currents and/or applying voltages to the conductive layer (5, 6) of the electrode element (B).

### 3.1 Electrode system

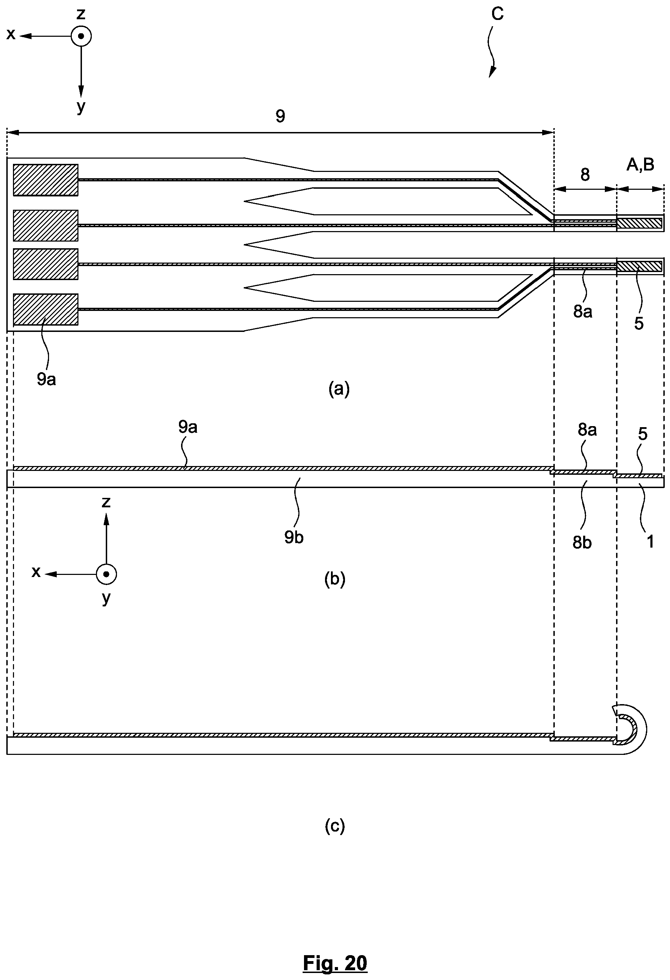

Figs. 20(a) and 20(b) schematically show a top view and a side view of an electrode system (C), respectively, according to an embodiment. The electrode system (C) comprises at least one electrode element (B) at one end of the electrode system (C) in the x-direction. The electrode system (C) further comprises a first connection portion (8) extending from the electrode element (B) and a second connection portion (9) extending from the first connection portion (8). The first and second connection portions (8, 9) comprise a first and second base layers (8b, 9b), respectively, each of which is preferably obtained by curing the resin mixture (10). More specifically, the substrate base layer (1) of the electrode element (B) (i.e. of the substrate (A)), the first and second base layers (8b, 9b) are obtained by curing the same resin mixture through photopolymerization process. The first base layer (8b) extends from the polymer compound layer (1) of the electrode element (B) in the x- and y-directions (and/or in the xy-plane), and the second base layer further extends from the first base layer in the x- and y-directions (and/or in the xy-plane).

Preferably, the first base layer (8b) has a higher cross-linking density than that of the substrate base layer (1) of the substrate (A), and the second base layer (9b) has a higher cross-linking density than the first base layer (8b). The first base layer may have a lower elasticity (and a higher stiffness) than the polymer compound layer (1) (or at least than the second surface (1-2) and/or the second sub-region (R₂)). The second base layer (9b) may further have a lower elasticity (and a higher stiffness) than the first base layer (8b), and the second base layer (9b) has accordingly the highest stiffness or hardness among these three polymer layers (1, 8b, 9b). The thickness of the first base layer (8b) in the z-direction may be therefore larger than the thickness d₁ of the substrate base layer (1), and the thickness of the second base layer (9b) in the z-direction may be larger than the thickness of the first base layer (8b). The cross-linking density and/or the thickness of each of the first and second base layers (8b, 9b) may be controlled such that a folding by liquid is induced substantially only at the part of the electrode element (B), whereas the mechanical structure of the first and second connection portions (8, 9) remains substantially unchanged. The first connection portion (8) having a higher elasticity and more flexible than the second connection portion (9) may be further advantageous in more accurate handling and positioning of the electrode system (C) during an implantation.

The first and second connection portions (8, 9) further comprise a conductive layer(s) (8a, 9a), which is deposited on the first and second base layers (8b, 9b). The conductive layer (8a) on the first base layer (8b) is physically and electrically connected to the conductive layer (5) of the electrode element (B), and would serve as a feed line. The conductive layer (8a) further extends to the surface of the second base layer (9b) of the second portion (9) and may include a contact pad for a connection to further instruments. In Fig. 20(a), two feed lines (8a) are arranged for each of the conductive layers (5) of the electrode element (B), which may be advantageous for e.g. calibrations, however, only a single feed line may be arranged for each of the conductive layers (5). A surface of the feed lines (8a, 9a) is preferably passivated by an insulating material.

Fig. 20(c) schematically illustrates the electrode system (C) with the electrode element (B) with the conductive layer (5) deposited on the second surface (1-2) of the substrate base layer (1) in a folded-state, which is induced by submerging the electrode element (B) into liquid. Alternatively, the third and/or fourth electrode element (B), in which the conductive layer (6) is deposited on the side of the first surface (1-1) of the substrate base layer (1), may be integrated into the electrode system (C). In such a case, an electrical and/or mechanical contact between a biological sample (which is to be positioned inside of the folded electrode element (B)) and the conductive layer (6) would be established through the protruding parts (3) and/or the through-hole (7). The surface of the conductive layer (6), which is deposited on the first layer (1-1) of the polymer compound layer (1) and corresponds to an outside of the folded electrode element (B) may be passivated by an insulating material. The conductive layer (8a, 9a) of the connection portions (8, 9) may be further formed on the side of the first layer (1-1).

The substrate base layer (1), the first base layer (8b) and the second base layer (9b) may be integrally formed by photopolymerization of the same resin mixture. The resin mixture (10) is at first casted on a glass substrate (S), and the mixture (10) is cured by irradiating light through the glass substrate (S). The curing condition, for example a time length of exposing the mixture with light and/or deposited light dose and/or irradiance of light, may be different at each of the substrate base layer (1), the first base layer (8b) and the second base layer (9b). At the part of the substrate base layer (1), the (frontal) photopolymerization process as described for any one of the first to fourth embodiments may be performed. The first base layer (8b) may be obtained by exposing the mixture (10) with light at a position being directly adjacent to the substrate base layer (1), for example by applying a longer time length of exposing the mixture with light and/or by depositing a higher light dose and/or by applying a higher irradiance of light than that of the substrate base layer (1). The second base layer (9b) may be obtained by exposing the mixture (10) with light at a position being directly adjacent to the first baye layer (8b) for example by applying a longer time length of exposing the mixture with light and/or by depositing a higher light dose and/or by applying a higher irradiance of light than that of the first base layer (1). All conductive layers (5, 8a, 9a) may be simultaneously formed, for example by metal sputtering process.

### 3.2 Example 7

An electrode system example was formed with the same resin mixture used for Example 1. The resin mixture was cured with different conditions at three different positions. At the part for being the substrate base layer (1), the mixture was cured by the frontal photopolymerization process as described in Example 1. At the part for being the first base layer (8b), the mixture was exposed with light having irradiance 9.3 × 10 ⁻¹ mW cm⁻² for 15 sec. At the part for being the second base layer (9b), the mixture was exposed with light having irradiance 3.1 mW cm⁻² for 10 sec. The obtained substrate base layer (1) had a thickness of 15 µm, the first base layer (8b) had a thickness of 30 µm, the second base layer (9b) had a thickness of 50 µm. A gold conductive layer was subsequently deposited on each of the substrate base layer (1), the first base layer (8b) and the second base layer (9b) by a lithography process and sputtering process, so that the conductive layer (5) on the substrate base layer (1), feedlines (8a) on the first base layer (8b) and contact pads (9a) on the second base layer (9a) are formed. The gold conductive layer had a thickness of 100 nm.

The part of the electrode element (B) of the obtained electrode system was submerged into saline solution (147 mM NaCl, 10 mM KCl, 4 mM CaCl2, 3 mM NaOH and 10 mM HEPES buffer), and the electrode element part was folded by the self-folding process of the substrate base layer (1) as described in the first embodiment. Chronoamperometry (CA) and impedance spectroscopy (EIS) were performed to characterize the folded conductive layer using a two-electrode configuration vs. open circuit potential (OCP). Both measurements were done in the saline solution (147 mM NaCl, 10 mM KCl, 4 mM CaCl2, 3 mM NaOH and 10 mM HEPES buffer). One electrode was set as the working electrode and the other was set as the combined reference and counter electrode. Voltage steps of 50 mV, 1 V and 3 V were applied in the CA measurement, after a stabilization period of 1 s to OCP. The EIS measurement was performed between 5 Hz and 500 kHz, with a sinusoidal signal of 10 mV of amplitude. Cyclic voltammetry scans were applied between -1 V and 1 V at 10 mV/s for 10 cycles to precondition the surface of the electrodes before each CA and EIS measurement. The measurement results show that there is no significant change in the cyclic voltametric curve with multiple scanning, which confirms that there was no delamination in the conductive layer of the electrode element through the folding process.

### List of reference signs

A: substrate, 1: substrate base layer, 1-1: first surface, 1-2: second surface, 2: band portion, 3: protruding part, B: electrode element, 5: electrically conductive layer, 6: electrically conductive layer, 7: dent portion, C: electrode system, 8: first connection portion, 8a: electrically conductive layer (feed lines), 8b: first base layer, 9: second connection portion, 9a: electrically conductive layer, 10: mixture, S: support

## Claims

1. A substrate (A) for implantable microelectrodes comprising
a substrate base layer (1) being a polymer compound layer,
wherein the substrate base layer (1) has a thickness (d₁) in the z-direction and comprises a first surface (1-1) and a second surface (1-2), each of the first surface (1-1) and the second surface (1-2) extending in the x-direction and the y-direction, the second surface (1-2) being opposite to the first surface (1-1) in the z-direction,
wherein the substrate base layer (1) consists of a hybrid material of a hydrophobic part and a hydrophilic part,
wherein the hybrid material is based on a mixture, the mixture comprising
(a) a photoinitiator being able to be activated by exposure to light,
(b) a first component comprising a first hydrophobic monomer, a first hydrophobic oligomer, a first hydrophobic polymer or a first hydrophobic copolymer,
wherein the first hydrophobic monomer or oligomer or polymer or copolymer of the first component comprises a first functional group,
wherein the first functional group is able to be activated by the activated photoinitiator, so that the first component is able to be polymerized through the first functional groups,
(c) a second component comprising a hydrophilic monomer or a hydrophilic oligomer or a hydrophilic polymer or a hydrophilic copolymer,
wherein the hydrophilic monomer or oligomer or polymer or copolymer comprises a second functional group,
wherein the second functional group is able to be activated by the activated photoinitiator, so that the second component is able to be polymerized through the second functional groups, and so that the first component and the second component are able to be co-polymerized through the first functional groups and the second functional groups, and
wherein the molecular weight (M_{wa}) of the first component is larger than the molecular weight (M_{wb}) of the second component,
wherein the first surface (1-1) is more hydrophilic than the second surface (1-2).

2. The substrate (A) according to claim 1,
wherein the substrate base layer (1) comprises a first sub-region (R₁) including the first surface (1-1) and a second sub-region (R₂) including the second surface (1-2), the first sub-region (R₁) being different from the second sub-region (R₂), wherein the first sub-region (R₁) is separated from the second sub-region (R₂) in the z-direction, wherein the first sub-region (R₁) has a higher swelling capacity than the second sub-region (R₂).

3. The substrate (A) according to any one of the preceding claims,
wherein the substrate base layer (1) further comprises a band portion (2), the band portion (2) having a higher cross-linking density than the second surface (1-2) and/or the second sub-region (R₂),
wherein the substrate base layer (1) has a length (l₁) in the x-direction and a width (w₁) in the y-direction,
wherein the band portion (2) extends for at least 50 % of the width (w₁) of the substrate base layer (1) in the y-direction,
wherein the band portion (2) extends for at least 50 % of the entire extent of the substrate base layer (1) in the z-direction,
wherein the band portion (2) has a thickness (l₂) in the x-direction, the thickness (l₂) being smaller than the length (l₁) of the layer (1) in the x-direction,
wherein the length (l₁) of the substrate base layer (1) is longer than the width (w₁) of the substrate base layer (1), and
- wherein the band portion (2) extends parallel to the y-direction,
or
- wherein the band portion (2) forms an angle with the y-direction, wherein the angle is more than 0° and less than or equal to 90°,
wherein the substrate base layer (1) comprises a plurality of the band portions (2), and the plurality of the band portions (2) is spaced apart from each other in the x-direction.

4. The substrate (A) according to any one of the preceding claims,
wherein the substrate base layer (1) is obtained by frontal photopolymerization of the mixture, and the first surface (1-1) is produced on the side from which the light is irradiated,
and/or
wherein the substrate base layer (1) comprises a dent portion (7) at the second surface (1-2) or at the first surface (1-1) of the substrate base layer (1), and wherein the dent portion (7) is a through-hole extending in the z-direction through the substrate base layer (1).

5. The substrate (A) according to any one of the preceding claims,
wherein the mixture further comprises
(d) a third component comprising a second hydrophobic monomer or a second hydrophobic oligomer or a second hydrophobic polymer or a second hydrophobic copolymer,
wherein the second hydrophobic monomer or oligomer or polymer or copolymer is different from the first hydrophobic monomer or oligomer or polymer or copolymer,
wherein the second hydrophobic monomer or oligomer or polymer or copolymer of the third component comprises a third functional group, the third functional group being a different end-group than the first functional group and/or the second functional group,
wherein the third functional group is able to be activated by the activated photoinitiator, so that the third component is able to be polymerized through the third functional groups, and/or so that the first component and the second component and the third component are able to be co-polymerized through the first functional groups and the second functional groups and the third functional groups,
and/or
(e) one or more further components selected from a group including
- diluent or buffer components,
- free radical quenchers,
- photosensitizers or UV absorbers
- cross-linkers.

6. The substrate (A) according to any one of the preceding claims,
wherein the substrate (A) further comprises a protruding part (3) protruding from the second surface (1-2) and/or the first surface (1-1) of the substrate base layer (1) in the z-direction,
wherein the protruding part (3) is rod-shaped and extends through the substrate base layer (1) in the z-direction, or
wherein the protruding part (3) is based on the mixture and is integrally formed with the substrate base layer (1),
wherein the protruding part (3) is at least partially electrically conductive.

7. The substrate (A) for according to any one of the preceding claims,
wherein the substrate (A) further comprises an embedded part being embedded in the layer (1),
wherein the embedded part is partially exposed to the outside of the substrate base layer (1).

8. The substrate (A) according to claim 7,
wherein the embedded part is rod-shaped and extends in the z-direction of the substrate base layer (1), and the embedded part has a first end and a second end, the second end being different from the first end,
wherein the first end is embedded inside of the substrate base layer (1) and the second end is exposed to the outside of the substrate base layer (1), and the second end is disposed within the plane of the second surface (1-2) or between the second surface (1-2) and the first surface (1-1),
or
wherein the embedded part further extends through the substrate base layer (1) and the first end is exposed to the outside of the layer, and the first end is disposed within the plane of the first surface (1-1) or between the second surface (1-2) and the first surface (1-1).

9. An electrode element (B) comprising the substrate (A) according to any one of claims 1 to 5,
wherein the electrode element (B) comprises an electrically conductive layer (5, 6) deposited on at least a part of the first surface (1-1) and/or a part of the second surface (1-2) of the substrate base layer (1).

10. An electrode element (B) comprising the substrate (A) according to any one of claims 6 to 8,
wherein the electrode element (B) comprises an electrically conductive layer (5, 6) deposited on at least a part of the second surface (1-2) or a part of the first surface (1-1) of the substrate base layer (1),
wherein the electrically conductive layer (5, 6) is further deposited on the protruding part (3) and/or on the embedded part (4).

11. The electrode element (B) according to claims 9 or 10,
wherein the electrode element (B) further comprises a further protruding part protruding from the electrically conductive layer (5, 6) in the z-direction, and the further protruding part is electrically conductive,
and/or
wherein the electrically conductive layer (5, 6) is transparent with respect to at least a range of light wavelengths.

12. An electrode system (C) comprising the electrode element (B) according to any one of claims 9 to 11,
wherein the electrode system (C) further comprises a first connection portion (8) extending from the electrode element (B) in the x- and y-direction,
wherein the first connection portion (8) comprises a first base layer (8b) extending from the substrate base layer (1) of the electrode element (B) and being integrally formed with the substrate base layer (1) of the electrode element (B), wherein the first base layer (8b) is based on the mixture,
wherein the first connection portion (8) comprises a further electrically conductive layer (8a) being deposited on the first base layer (8b) and being connected to the electrically conductive layer (5) of the electrode element (B),
wherein the first base layer (8b) has a higher cross-linking density than the substrate base layer (1) of the electrode element (B), and
wherein the first base layer (8b) has a larger thickness in the z-direction than the thickness (d₁) of the substrate base layer (1).

13. A method for manufacturing a substrate (A) according to any one of claims 1 to 8, the method comprising
disposing the mixture on a surface of a support (S),
exposing the mixture with light, thereby the first surface (1-1) of the substrate base layer (1) is obtained on the side from which the light is irradiated, and the second surface (1-2) of the layer is obtained on the side opposite to the first surface in the z-direction.

14. The method according to claim 13 further comprising
forming the band portion (2) by exposing only at a part of the mixture (10) and/or the substrate base layer (1) on the support (S) with light,
- for a time length longer than a time length applied for the other part of the substrate base layer (1), and/or
- wherein said part is exposed with light having an irradiance higher than an irradiance applied for the other part of the substrate base layer (1),
so that the band portion (2) has a higher cross-linking density than the other part,
and/or
forming the dent portion (7) by irradiating a laser beam onto a part of the first surface (1-1) or onto the second surface (1-2) of the layer (1).

15. The method according to any one of claims 13 to 14 further comprising
depositing the protruding part (3) or the embedded part (4) on the surface of the support before the step of disposing of the mixture on the surface of the support (S),
or
depositing the protruding part (3) or the embedded part (4) on the first surface (1-1) and/or the second surface (1-2) of the layer (1) after the step of exposing of the mixture with light,
or
forming the protruding part (3) by further exposing only a part of the substrate base layer (1) with light in the mixture (10), so that the protruding part (3) protrudes from the first surface (1-1),
and/or
forming the protruding part (3) by exposing only a part of the substrate base layer (1) with light in the mixture with light having irradiance higher than irradiance applied for the other part of the substrate base layer (1), so that the protruding part (3) protrudes from the first surface (1-1),
and/or
wherein the mixture is exposed with light through the support (S), so that the first surface (1-1) is obtained at an interface between the mixture and the support (S),
or
wherein the mixture is exposed with the light from a side of the mixture relative to the interface between the support (S) and the mixture so that the second surface (1-2) is obtained at the interface between the mixture and the support (S).

## Patentansprüche

1. Substrat (A) für implantierbare Mikroelektroden, aufweisend
eine Substratbasisschicht (1), welche eine Polymerverbundschicht ist,
wobei die Substratbasisschicht (1) eine Dicke (d₁) in der z-Richtung aufweist und eine erste Fläche (1-1) und eine zweite Fläche (1-2) aufweist, wobei sich sowohl die erste Fläche (1-1) als auch die zweite Fläche (1-2) in der x-Richtung und in der y-Richtung erstrecken, wobei die zweite Fläche (1-2) der ersten Fläche (1-1) in der z-Richtung gegenüberliegt,
wobei die Substratbasisschicht (1) aus einem hybriden Material mit einem hydrophoben Teil und einem hydrophilen Teil besteht,
wobei das hybride Material auf einem Gemisch basiert, wobei das Gemisch aufweist:
(a) einen Photoinitiator, welcher in der Lage ist, durch Bestrahlung mit Licht aktiviert zu werden,
(b) eine erste Komponente, welche ein erstes hydrophobes Monomer, ein erstes hydrophobes Oligomer, ein erstes hydrophobes Polymer oder ein erstes hydrophobes Copolymer aufweist,
wobei das erste hydrophobe Monomer oder Oligomer oder Polymer oder Copolymer der ersten Komponente eine erste funktionelle Gruppe aufweist,
wobei die erste funktionelle Gruppe in der Lage ist, durch den aktivierten Photoinitiator aktiviert zu werden, so dass die erste Komponente in der Lage ist, durch die ersten funktionellen Gruppen polymerisiert zu werden,
(c) eine zweite Komponente, welche ein hydrophiles Monomer oder ein hydrophiles Oligomer oder ein hydrophiles Polymer oder ein hydrophiles Copolymer aufweist,
wobei das hydrophile Monomer oder Oligomer oder Polymer oder Copolymer eine zweite funktionelle Gruppe aufweist,
wobei die zweite funktionelle Gruppe in der Lage ist, durch den aktivierten Photoinitiator aktiviert zu werden, so dass die zweite Komponente in der Lage ist, durch die zweiten funktionellen Gruppen polymerisiert zu werden, und so dass die erste Komponente und die zweite Komponente in der Lage sind, durch die ersten funktionellen Gruppen und die zweiten funktionellen Gruppen copolymerisiert zu werden, und
wobei das Molekulargewicht (M_{wa}) der ersten Komponente höher ist als das Molekulargewicht (M_{wb}) der zweiten Komponente,
wobei die erste Fläche (1-1) stärker hydrophil ist als die zweite Fläche (1-2).

2. Substrat (A) nach Anspruch 1,
wobei die Substratbasisschicht (1) eine erste Teilregion (R₁) aufweist, welche die erste Fläche (1-1) umfasst, und eine zweite Teilregion (R₂) aufweist, welche die zweite Fläche (1-2) umfasst, wobei sich die erste Teilregion (R₁) von der zweiten Teilregion (R₂) unterscheidet, wobei die erste Teilregion (R₁) in der z-Richtung von der zweiten Teilregion (R₂) getrennt ist,
wobei die erste Teilregion (R₁) eine höhere Quellfähigkeit aufweist als die zweite Teilregion (R₂).

3. Substrat (A) nach einem der vorhergehenden Ansprüche,
wobei die Substratbasisschicht (1) ferner einen Bandabschnitt (2) aufweist, wobei der Bandabschnitt (2) eine höhere Vernetzungsdichte aufweist als die zweite Fläche (1-2) und/oder die zweite Teilregion (R₂),
wobei die Substratbasisschicht (1) eine Länge (I₁) in der x-Richtung und eine Breite (w₁) in der y-Richtung aufweist,
wobei sich der Bandabschnitt (2) in der y-Richtung über mindestens 50 % der Breite (w₁) der Substratbasisschicht (1) erstreckt,
wobei sich der Bandabschnitt (2) in der z-Richtung über mindestens 50 % des gesamten Ausmaßes der Substratbasisschicht (1) erstreckt,
wobei der Bandabschnitt (2) eine Dicke (l₂) in der x-Richtung aufweist, wobei die Dicke (I₂) geringer ist als die Länge (I₁) der Schicht (1) in der x-Richtung,
wobei die Länge (I₁) der Substratbasisschicht (1) größer ist als die Breite (w₁) der Substratbasisschicht (1), und
- wobei sich der Bandabschnitt (2) parallel zur y-Richtung erstreckt,
oder
- wobei der Bandabschnitt (2) einen Winkel mit der y-Richtung bildet, wobei der Winkel größer als 0° und kleiner oder gleich 90° ist,
wobei die Substratbasisschicht (1) mehrere der Bandabschnitte (2) aufweist und die mehreren der Bandabschnitte (2) in der x-Richtung einen Abstand voneinander aufweisen.

4. Substrat (A) nach einem der vorhergehenden Ansprüche,
wobei die Substratbasisschicht (1) durch frontale Photopolymerisation des Gemisches erhalten wird und die erste Fläche (1-1) auf der Seite hergestellt wird, von welcher das Licht eingestrahlt wird,
und/oder
wobei die Substratbasisschicht (1) einen Zahnabschnitt (7) an der zweiten Fläche (1-2) oder an der ersten Fläche (1-1) der Substratbasisschicht (1) aufweist und wobei der Zahnabschnitt (7) ein Durchgangsloch ist, welches sich in der z-Richtung durch die Substratbasisschicht (1) erstreckt.

5. Substrat (A) nach einem der vorhergehenden Ansprüche,
wobei das Gemisch ferner aufweist:
(d) eine dritte Komponente, welche ein zweites hydrophobes Monomer oder ein zweites hydrophobes Oligomer oder ein zweites hydrophobes Polymer oder ein zweites hydrophobes Copolymer aufweist,
wobei sich das zweite hydrophobe Monomer oder Oligomer oder Polymer oder Copolymer von dem ersten Monomer oder Oligomer oder Polymer oder Copolymer unterscheidet,
wobei das zweite hydrophobe Monomer oder Oligomer oder Polymer oder Copolymer der dritten Komponente eine dritte funktionelle Gruppe aufweist, wobei die dritte funktionelle Gruppe eine andere Endgruppe als die erste funktionelle Gruppe und/oder die zweite funktionelle Gruppe ist,
wobei die dritte funktionelle Gruppe in der Lage ist, durch den aktivierten Photoinitiator aktiviert zu werden, so dass die dritte Komponente in der Lage ist, durch die dritten funktionellen Gruppen polymerisiert zu werden, und/oder so dass die erste Komponente und die zweite Komponente und die dritte Komponente in der Lage sind, durch die ersten funktionellen Gruppen und die zweiten funktionellen Gruppen und die dritten funktionellen Gruppen copolymerisiert zu werden,
und/oder
(e) eine oder mehrere weitere Komponenten, ausgewählt aus einer Gruppe, umfassend
- Verdünnungs- oder Pufferkomponenten,
- freie Radikal-Quencher,
- Photosensibilisatoren oder UV-Absorber
- Vernetzungsmittel.

6. Substrat (A) nach einem der vorhergehenden Ansprüche,
wobei das Substrat (A) ferner einen vorstehenden Teil (3) aufweist, welcher in der z-Richtung von der zweiten Fläche (1-2) und/oder der ersten Fläche (1-1) der Substratbasisschicht (1) vorsteht,
wobei der vorstehende Teil (3) stabförmig ist und sich in der z-Richtung durch die Substratbasisschicht (1) erstreckt, oder
wobei der vorstehende Teil (3) auf dem Gemisch basiert und einstückig mit der Substratbasisschicht (1) ausgebildet ist,
wobei der vorstehende Teil (3) zumindest teilweise elektrisch leitfähig ist.

7. Substrat (A) nach einem der vorhergehenden Ansprüche,
wobei das Substrat (A) ferner einen eingebetteten Teil aufweist, welcher in die Schicht (1) eingebettet ist;
wobei der eingebettete Teil teilweise zur Außenseite der Substratbasisschicht (1) frei liegt.

8. Substrat (A) nach Anspruch 7,
wobei der eingebettete Teil stabförmig ist und sich in der z-Richtung der Substratbasisschicht (1) erstreckt und der eingebettete Teil ein erstes Ende und ein zweites Ende aufweist, wobei sich das zweite Ende von dem ersten Ende unterscheidet,
wobei das erste Ende innerhalb der Substratbasisschicht (1) eingebettet ist und das zweite Ende zur Außenseite der Substratbasisschicht (1) frei liegt und das zweite Ende innerhalb der Ebene der zweiten Fläche (1-2) oder zwischen der zweiten Fläche (1-2) und der ersten Fläche (1-1) angeordnet ist,
oder
wobei sich der eingebettete Teil ferner durch die Substratbasisschicht (1) erstreckt und das erste Ende zur Außenseite der Schicht frei liegt und das erste Ende innerhalb der Ebene der ersten Fläche (1-1) oder zwischen der zweiten Fläche (1-2) und der ersten Fläche (1-1) angeordnet ist.

9. Elektrodenelement (B), welches das Substrat (A) nach einem der Ansprüche 1 bis 5 aufweist,
wobei das Elektrodenelement (B) eine elektrisch leitfähige Schicht (5, 6) aufweist, die zumindest auf einem Teil der ersten Fläche (1-1) und/oder einem Teil der zweiten Fläche (1-2) der Substratbasisschicht (1) abgeschieden ist.

10. Elektrodenelement (B), welches das Substrat (A) nach einem der Ansprüche 6 bis 8 aufweist,
wobei das Elektrodenelement (B) eine elektrisch leitfähige Schicht (5, 6) aufweist, die zumindest auf einem Teil der zweiten Fläche (1-2) oder einem Teil der ersten Fläche (1-1) der Substratbasisschicht (1) abgeschieden ist,
wobei die elektrisch leitfähige Schicht (5, 6) ferner auf dem vorstehenden Teil (3) und/oder auf dem eingebetteten Teil (4) abgeschieden ist.

11. Elektrodenelement (B) nach Anspruch 9 oder 10,
wobei das Elektrodenelement (B) ferner einen weiteren vorstehenden Teil aufweist, welcher in der z-Richtung von der elektrisch leitfähigen Schicht (5, 6) vorsteht, und der weitere vorstehende Teil elektrisch leitfähig ist,
und/oder
wobei die elektrisch leitfähige Schicht (5, 6) für zumindest einen Bereich von Lichtwellenlängen transparent ist.

12. Elektrodensystem (C), welches das Elektrodenelement (B) nach einem der Ansprüche 9 bis 11 aufweist,
wobei das Elektrodensystem (C) ferner einen ersten Verbindungsabschnitt (8) aufweist, welcher sich in der x- und der y-Richtung von dem Elektrodenelement (B) erstreckt,
wobei der erste Verbindungsabschnitt (8) eine erste Basisschicht (8b) aufweist, welche sich von der Substratbasisschicht (1) des Elektrodenelements (B) erstreckt und einstückig mit der Substratbasisschicht (1) des Elektrodenelements (B) ausgebildet ist, wobei die erste Basisschicht (8b) auf dem Gemisch basiert,
wobei der erste Verbindungsabschnitt (8) eine weitere elektrisch leitfähige Schicht (8a) aufweist, welche auf der ersten Basisschicht (8b) abgeschieden ist und mit der elektrisch leitfähigen Schicht (5) des Elektrodenelements (B) verbunden ist,
wobei die erste Basisschicht (8b) eine höhere Vernetzungsdichte aufweist als die Substratbasisschicht (1) des Elektrodenelements (B), und
wobei die erste Basisschicht (8b) in der z-Richtung eine größere Dicke aufweist als die Dicke (d₁) der Substratbasisschicht (1).

13. Verfahren zum Herstellen eines Substrats (A) nach einem der Ansprüche 1 bis 8, wobei das Verfahren umfasst:
Abscheiden des Gemisches auf einer Fläche eines Trägers (S),
Bestrahlen des Gemisches mit Licht, wodurch die erste Fläche (1-1) der Substratbasisschicht (1) auf der Seite erhalten wird, von welcher das Licht eingestrahlt wird, und die zweite Fläche (1-2) der Schicht in der z-Richtung auf der Seite gegenüber der ersten Fläche erhalten wird.

14. Verfahren nach Anspruch 13, ferner umfassend
Bilden des Bandabschnitts (2) durch Bestrahlen nur eines Teils des Gemisches (10) und/oder der Substratbasisschicht (1) auf dem Träger (S) mit Licht,
- für eine Zeit, die länger ist als eine Zeit, die für den anderen Teil der Substratbasisschicht (1) angewendet wird, und/oder
- wobei der Teil mit Licht bestrahlt wird, das eine höhere Bestrahlungsstärke aufweist als die Bestrahlungsstärke, die für den anderen Teil der Substratbasisschicht (1) angewendet wird,
so dass der Bandabschnitt (2) eine höhere Vernetzungsdichte als der andere Teil aufweist,
und/oder
Bilden des Zahnabschnitts (7) durch Strahlen eines Laserstrahls auf einen Teil der ersten Fläche (1-1) oder auf die zweite Fläche (1-2) der Schicht (1).

15. Verfahren nach einem der Ansprüche 13 bis 14, ferner umfassend
Abscheiden des vorstehenden Teils (3) oder des eingebetteten Teils (4) auf der Fläche des Trägers vor dem Schritt des Abscheidens des Gemisches auf der Fläche des Trägers (S),
oder
Abscheiden des vorstehenden Teils (3) oder des eingebetteten Teils (4) auf der ersten Fläche (1-1) und/oder der zweiten Fläche (1-2) der Schicht (1) nach dem Schritt des Bestrahlens des Gemisches mit Licht,
oder
Bilden des vorstehenden Teils (3) durch weiteres Bestrahlen nur eines Teils der Substratbasisschicht (1) in dem Gemisch (10) mit Licht, so dass der vorstehende Teil (3) von der ersten Fläche (1-1) vorsteht,
und/oder
Bilden des vorstehenden Teils (3) durch Bestrahlen nur eines Teils der Substratbasisschicht (1) in dem Gemisch mit Licht, welches eine Bestrahlungsstärke aufweist, die höher ist als eine Bestrahlungsstärke, die für den anderen Teil der Substratbasisschicht (1) angewendet wird, so dass der vorstehende Teil (3) von der ersten Fläche (1-1) vorsteht,
und/oder
wobei das Gemisch durch den Träger (S) hindurch mit Licht bestrahlt wird, so dass die erste Fläche (1-1) an einer Grenzfläche zwischen dem Gemisch und dem Träger (S) erhalten wird,
oder
wobei das Gemisch in Bezug auf die Grenzfläche zwischen dem Träger (S) und dem Gemisch von einer Seite des Gemisches mit dem Licht bestrahlt wird, so dass die zweite Fläche (1-2) an der Grenzfläche zwischen dem Gemisch und dem Träger (S) erhalten wird.

## Revendications

1. Substrat (A) pour microélectrodes implantables comprenant
une couche de base de substrat (1) qui est une couche de composé polymère,
dans lequel la couche de base de substrat (1) possède une épaisseur (d₁) dans la direction z et comprend une première surface (1-1) et une deuxième surface (1-2), chacune de la première surface (1-1) et de la deuxième surface (1-2) s'étendant dans la direction x et la direction y, la deuxième surface (1-2) étant opposée à la première surface (1-1) dans la direction z,
dans lequel la couche de base de substrat (1) est constituée d'un matériau hybride composé d'une partie hydrophobe et d'une partie hydrophile,
dans lequel le matériau hybride est basé sur un mélange comprenant
(a) un photo-initiateur pouvant être activé par exposition à la lumière,
(b) un premier composant comprenant un premier monomère hydrophobe, un premier oligomère hydrophobe, un premier polymère hydrophobe ou un premier copolymère hydrophobe,
dans lequel le premier monomère ou oligomère ou polymère ou copolymère hydrophobe du premier composant comprend un premier groupe fonctionnel, dans lequel le premier groupe fonctionnel peut être activé par le photo-initiateur activé, de sorte que le premier composant peut être polymérisé par le biais des premiers groupes fonctionnels,
(c) un deuxième composant comprenant un monomère hydrophile ou un oligomère hydrophile ou un polymère hydrophile ou un copolymère hydrophile,
dans lequel le monomère ou l'oligomère ou le polymère ou le copolymère hydrophile comprend un deuxième groupe fonctionnel,
dans lequel le deuxième groupe fonctionnel peut être activé par le photo-initiateur activé, de sorte que le deuxième composant peut être polymérisé par le biais des deuxièmes groupes fonctionnels, et de sorte que le premier composant et le deuxième composant peuvent être copolymérisés par le biais des premiers groupes fonctionnels et des deuxièmes groupes fonctionnels, et
dans lequel la masse moléculaire (M_{wa}) du premier composant est supérieure à la masse moléculaire (M_{wb}) du deuxième composant,
dans lequel la première surface (1-1) est plus hydrophile que la deuxième surface (1-2).

2. Substrat (A) selon la revendication 1,
dans lequel la couche de base de substrat (1) comprend une première sous-région (R₁) comprenant la première surface (1-1) et une deuxième sous-région (R₂) comprenant la deuxième surface (1-2), la première sous-région (R₁) étant différente de la deuxième sous-région (R₂), dans lequel la première sous-région (R₁) est séparée de la deuxième sous-région (R₂) dans la direction z,
dans lequel la première sous-région (R₁) possède une capacité de gonflement supérieure à la deuxième sous-région (R₂).

3. Substrat (A) selon l'une quelconque des revendications précédentes,
dans lequel la couche de base de substrat (1) comprend en outre une partie de bande (2), la partie de bande (2) ayant une densité de réticulation supérieure à celle de la deuxième surface (1-2) et/ou de la deuxième sous-région (R₂),
dans lequel la couche de base de substrat (1) possède une longueur (l₁) dans la direction x et une largeur (w₄) dans la direction y,
dans lequel la partie de bande (2) s'étend sur au moins 50 % de la largeur (w₁) de la couche de base de substrat (1) dans la direction y,
dans lequel la partie de bande (2) s'étend sur au moins 50 % de l'étendue totale de la couche de base de substrat (1) dans la direction z,
dans lequel la partie de bande (2) possède une épaisseur (l₂) dans la direction x, l'épaisseur (l₂) étant inférieure à la longueur (l₁) de la couche (1) dans la direction x,
dans lequel la longueur (l₁) de la couche de base de substrat (1) est supérieure à la largeur (w₁) de la couche de base de substrat (1), et
- dans lequel la partie de bande (2) s'étend parallèlement à la direction y,
ou
- dans lequel la partie de bande (2) forme un angle avec la direction y, dans lequel l'angle est supérieur à 0° et inférieur ou égal à 90°,
dans lequel la couche de base de substrat (1) comprend une pluralité de parties de bande (2), et la pluralité de parties de bande (2) sont espacées les unes des autres dans la direction x.

4. Substrat (A) selon l'une quelconque des revendications précédentes,
dans lequel la couche de base de substrat (1) est obtenue par photopolymérisation frontale du mélange, et la première surface (1-1) est produite sur lu côté d'où la lumière est irradiée,
et/ou
dans lequel la couche de base de substrat (1) comprend une partie bosselée (7) au niveau de la deuxième surface (1-2) ou au niveau de la première surface (1-1) de la couche de base de substrat (1), et dans lequel la partie bosselée (7) est un trou traversant s'étendant dans la direction z à travers la couche de base de substrat (1).

5. Substrat (A) selon l'une quelconque des revendications précédentes,
dans lequel le mélange comprend en outre
(d) un troisième composant comprenant un deuxième monomère hydrophobe ou un deuxième oligomère hydrophobe ou un deuxième polymère hydrophobe ou un deuxième copolymère hydrophobe,
dans lequel le deuxième monomère ou oligomère ou polymère ou copolymère hydrophobe est différent du premier monomère ou oligomère ou polymère ou copolymère hydrophobe,
dans lequel le deuxième monomère ou oligomère ou polymère ou copolymère hydrophobe du troisième composant comprend un troisième groupe fonctionnel, le troisième groupe fonctionnel étant un groupe terminal différent du premier groupe fonctionnel et/ou du deuxième groupe fonctionnel,
dans lequel le troisième groupe fonctionnel peut être activé par le photo-initiateur activé, de sorte que le troisième composant peut être polymérisé par le biais des troisièmes groupes fonctionnels, et/ou de sorte que le premier composant et le deuxième composant et le troisième composant peuvent être copolymérisés par le biais des premiers groupes fonctionnels et des deuxièmes groupes fonctionnels et des troisièmes groupes fonctionnels,
et/ou
(e) un ou plusieurs composants supplémentaires choisis dans un groupe comprenant
- des composants diluants ou tampons ;
- des piégeurs de radicaux libres,
- des photosensibilisateurs ou absorbeurs d'UV
- des agents de réticulation.

6. Substrat (A) selon l'une quelconque des revendications précédentes,
dans lequel le substrat (A) comprend en outre une partie saillante (3) faisant saillie de la deuxième surface (1-2) et/ou de la première surface (1-1) de la couche de base de substrat (1) dans la direction z,
dans lequel la partie saillante (3) est en forme de tige et s'étend à travers la couche de base de substrat (1) dans la direction z, ou
dans lequel la partie saillante (3) est basée sur le mélange et est formée d'un seul tenant avec la couche de base de substrat (1),
dans lequel la partie saillante (3) est au moins partiellement électriquement conductrice.

7. Substrat (A) selon l'une quelconque des revendications précédentes,
dans lequel le substrat (A) comprend en outre une partie incorporée qui est incorporée dans la couche (1),
dans lequel la partie incorporée est partiellement exposée à l'extérieur de la couche de base de substrat (1).

8. Substrat (A) selon la revendication 7,
dans lequel la partie incorporée est en forme de tige et s'étend dans la direction z de la couche de base de substrat (1), et la partie incorporée possède une première extrémité et une deuxième extrémité, la deuxième extrémité étant différente de la première extrémité,
dans lequel la première extrémité est incorporée à l'intérieur de la couche de base de substrat (1) et la deuxième extrémité est exposée à l'extérieur de la couche de base de substrat (1), et la deuxième extrémité est déposée dans le plan de la deuxième surface (1-2) ou entre la deuxième surface (1-2) et la première surface (1-1),
ou
dans lequel la partie incorporée s'étend en outre à travers la couche de base de substrat (1) et la première extrémité est exposée à l'extérieur de la couche, et la première extrémité est déposée dans le plan de la première surface (1-1) ou entre la deuxième surface (1-2) et la première surface (1-1).

9. Élément d'électrode (B) comprenant le substrat (A) selon l'une quelconque des revendications 1 à 5,
dans lequel l'élément d'électrode (B) comprend une couche électriquement conductrice (5, 6) déposée sur au moins une partie de la première surface (1-1) et/ou une partie de la deuxième surface (1-2) de la couche de base de substrat (1).

10. Élément d'électrode (B) comprenant le substrat (A) selon l'une quelconque des revendications 6 à 8,
dans lequel l'élément d'électrode (B) comprend une couche électriquement conductrice (5, 6) déposée sur au moins une partie de la deuxième surface (1-2) ou une partie de la première surface (1-1) de la couche de base de substrat (1),
dans lequel la couche électriquement conductrice (5, 6) est en outre déposée sur la partie saillante (3) et/ou sur la partie incorporée (4).

11. Élément d'électrode (B) selon les revendications 9 ou 10,
dans lequel l'élément d'électrode (B) comprend en outre une partie saillante supplémentaire faisant saillie de la couche électriquement conductrice (5, 6) dans la direction z, et la partie saillante supplémentaire est électriquement conductrice,
et/ou
dans lequel la couche électriquement conductrice (5, 6) est transparente par rapport à au moins une plage de longueurs d'onde lumineuses.

12. Système d'électrode (C) comprenant l'élément d'électrode (B) selon l'une quelconque des revendications 9 à 11,
dans lequel le système d'électrode (C) comprend en outre une première partie de connexion (8) s'étendant depuis l'élément d'électrode (B) dans les directions x et y,
dans lequel la première partie de connexion (8) comprend une première couche de base (8b) s'étendant depuis la couche de base de substrat (1) de l'élément d'électrode (B) et étant formée d'un seul tenant avec la couche de base de substrat (1) de l'élément d'électrode (B),
dans lequel la première couche de base (8b) est basée sur le mélange,
dans lequel la première partie de connexion (8) comprend une couche électriquement conductrice supplémentaire (8a) qui est déposée sur la première couche de base (8b) et connectée à la couche électriquement conductrice (5) de l'élément d'électrode (B),
dans lequel la première couche de base (8b) possède une densité de réticulation supérieure à celle de la couche de base de substrat (1) de l'élément d'électrode (B), et
dans lequel la première couche de base (8b) possède une épaisseur dans la direction z supérieure à l'épaisseur (d₁) de la couche de base de substrat (1).

13. Procédé de fabrication d'un substrat (A) selon l'une quelconque des revendications 1 à 8, le procédé comprenant
le dépôt du mélange sur une surface d'un support (S),
l'exposition du mélange à la lumière, de cette façon la première surface (1-1) de la couche de base de substrat (1) est obtenue sur le côté d'où la lumière est irradiée, et la deuxième surface (1-2) de la couche est obtenue sur le côté opposé à la première surface dans la direction z.

14. Procédé selon la revendication 13 comprenant en outre
la formation de la partie de bande (2) par exposition uniquement au niveau d'une partie du mélange (10) et/ou de la couche de base de substrat (1) sur le support (S) à la lumière,
- pendant une durée supérieure à une durée appliquée à l'autre partie de la couche de base de substrat (1), et/ou
- dans lequel ladite partie est exposée à la lumière ayant une irradiance supérieure à une irradiance appliquée à l'autre partie de la couche de base de substrat (1),
de sorte que la partie de bande (2) possède une densité de réticulation supérieure à celle de l'autre partie,
et/ou
la formation de la partie bosselée (7) par irradiation d'un faisceau laser sur une partie de la première surface (1-1) ou sur la deuxième surface (1-2) de la couche (1).

15. Procédé selon l'une quelconque des revendications 13 à 14 comprenant en outre
le dépôt de la partie saillante (3) ou de la partie incorporée (4) sur la surface du support avant l'étape de dépôt du mélange sur la surface du support (S),
ou
le dépôt de la partie saillante (3) ou de la partie incorporée (4) sur la première surface (1-1) et/ou la deuxième surface (1-2) de la couche (1) après l'étape d'exposition du mélange à la lumière,
ou
la formation de la partie saillante (3) en exposant en outre uniquement une partie de la couche de base de substrat (1) à la lumière dans le mélange (10), de sorte que la partie saillante (3) fait saillie de la première surface (1-1),
et/ou
la formation de la partie saillante (3) en exposant uniquement une partie de la couche de base de substrat (1) à la lumière dans le mélange à une lumière ayant une irradiance supérieure à l'irradiance appliquée à l'autre partie de la couche de base de substrat (1), de sorte que la partie saillante (3) fait saillie de la première surface (1-1),
et/ou
dans lequel le mélange est exposé à la lumière à travers le support (S), de sorte que la première surface (1-1) est obtenue au niveau d'interface entre le mélange et le support (S),
ou
dans lequel le mélange est exposé à la lumière provenant d'un côté du mélange par rapport à l'interface entre le support (S) et le mélange de sorte que la deuxième surface (1-2) est obtenue au niveau de l'interface entre le mélange et le support (S).
